# FASCICULE DE BREVET EUROPEEN

(11) **EP 1 235 928 B1**
(45) Date de publication et mention de la délivrance du brevet: **13.10.2004**
(21) Numéro de dépôt: 00990049.9
(22) Date de dépôt: 11.12.2000
(51) Int. Cl.: C12Q 1/34, C12Q 1/04

(54) **NOUVEAUX SUBSTRATS CHROMOGENES A BASE D'ALZARINE**
ALIZARIN-DERIVATE ALS NEUE CHROMOGENE SUBSTRATE
NOVEL ALIZARIN-BASED CHROMOGENIC SUBSTRATES

(30) Priorité: 09.12.1999 FR 9915515
(43) Date de publication de la demande: 04.09.2002
(73) Titulaire: Biomerieux S.A., 69280 Marcy l'Etoile (FR)
(72) Inventeur: ARMSTRONG, Lyle, Ashington, Northumberland NE638 AE (GB); JAMES, Arthur, Jesmond, Newcastle Upon Tyne NE21 HR (GB)
(74) Mandataire: Bonneau, Gérard
(86) Numéro de dépôt international: PCT/FR2000/003465
(87) Numéro de publication internationale: WO 2001/042490

(56) Documents cités:
- WO-A-98/55644
- MASAWAKI, TERUYUKI ET AL: "Selective solvent extraction of ruberythric acid from madder roots and subsequent hydrolysis with.beta.- glucosidase" J. FERMENT. BIOENG. (1996), 81(6), 567-569, XP000938126 cité dans la demande
- VAN DER PLAS, LINUS H. W. ET AL: "Anthraquinone glycosylation and hydrolysis in Morinda citrifolia cell suspensions. Regulation and function" J. PLANT PHYSIOL. (1998), 152(2/3), 235-241, XP000938286 cité dans la demande
- MATEJU, J. ET AL: "Microbial glucosidation of dihydroxyanthraquinones. General properties of the glucosidation system" FOLIA MICROBIOL. (PRAGUE) (1974), 19(4), 307-16, XP000938150 cité dans la demande
- JAMES A L; PERRY J D; CHILVERS K; ROBSON I S; ARMSTRONG L; ORR K E: "Alizarin-beta-D-galactoside: A new substrate for the detection of bacterial beta-galactosidase" LETTERS IN APPLIED MICROBIOLOGY, vol. 30, avril 2000 (2000-04), pages 336-340, XP000938196

## Description

La présente invention concerne la mise en évidence d'enzymes de type hydrolase, en particulier d'osidase, d'estérase ou de peptidase, par l'utilisation de substrats chromogènes efficaces.

Depuis de très nombreuses années, on utilise des substrats particuliers pour permettre la détermination de la présence ou de l'absence d'activités enzymatiques caractéristiques de micro-organismes. Par le choix des substrats, selon qu'il y a réaction ou non, il est possible de caractériser la nature d'un genre de micro-organismes ou de différencier des souches et/ou des espèces d'un genre microbien donné.

Les substrats synthétiques d'enzymes sont constitués de deux parties, une première partie spécifique de l'activité enzymatique à révéler, ci-après appelée partie cible, et une seconde partie faisant office de marqueur, ci-après appelée partie marqueur.

Ces substrats particuliers peuvent être fluorescents ou chromogènes. En fait, c'est la seconde partie marqueur ou le produit de sa réaction avec un ou plusieurs autres composés, voir à ce sujet la demande de brevet PCT/FR99/00781 déposée au nom de la Demanderesse, qui est fluorescente ou chromogène, lorsqu'elle n'est plus associée à la première partie cible.

Dans le cas présent, il s'agit de substrats chromogènes à base d'Alizarines ou d'Anthrarobines qui, sous cette forme de substrats, sont généralement légèrement colorés. Néanmoins, la coloration due à la partie marqueur est renforcée et/ou modifiée par l'hydrolyse et donc la séparation de la partie cible par rapport à ladite partie marqueur. Préférentiellement, la présence d'un révélateur, tel qu'un sel de métal, un pH alcalin, améliore encore les caractéristiques colorées du produit obtenu.

La capacité des Alizarines à former des chélates métalliques colorés a été trouvée durant le dix-neuvième siècle. Dès 1826, quand les Alizarines ont été pour la première fois isolées à partir de la plante Rubia tinctorum, leur propriété de matière colorante a été utilisée dans la teinture des vêtements.

La synthèse globale des Alizarines a été décrite par Graebe et Liebermann en 1869. La même année, W. H. Perkin a élargi le nombre d'Alizarines qui peuvent être synthétisées, en proposant différents substituants sur les positions R₃, R₄, R₅, R₆, R₇ et R₈ du noyau Anthracène ci-après :

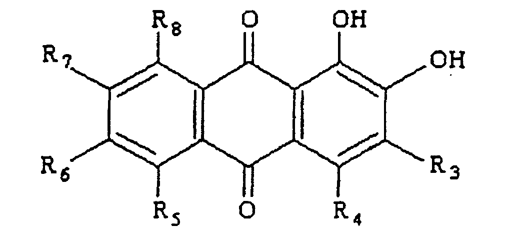

Les Alizarines ne sont pas des colorants per se mais forment des pigments insolubles avec des oxydes métalliques. Par exemple lorsque la position R₃ est substituée par un groupement sulfonique, la chélation avec un sel d'Aluminium donne une couleur rouge écarlate éclatante, alors que la chélation avec un sel de Chrome donne un rouge Bordeaux. Autre exemple, les 3-Nitroalizarine et 4-Nitroalizarine donnent des chélates de différentes colorations en fonction des sels métalliques qui leur sont associées. L'intérêt suscité par les Alizarines dans la teinture est en grande partie dû à la stabilité des chélates métalliques, ainsi formés, que ce soit aux savons, aux acides et aux hydroxydes de métal alcalin.

Parmi les dérivés d'Alizarine qui peuvent être aisément synthétisés, on note qu'à partir de 3-Nitroalizarine et 4-Nitroalizarine, on obtient des 3 Aminoalizarine et 4-Aminoalizarine. La 4-Aminoalizarine est représentée ci-dessous :

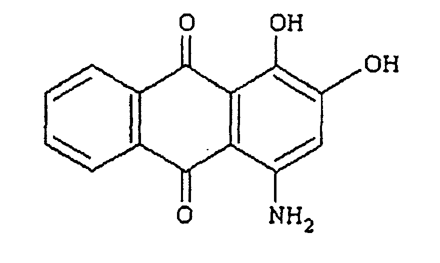

Cette molécule est particulièrement intéressante car elle donne une couleur pourpre en présence d'Aluminium. De plus, elle sert comme point de départ pour la synthèse, au moyen d'une réaction de Skraup bien connue de l'homme du métier, des Alizarines quinalines, dont un exemple, l'Alizarine α-quinoline de couleur verte, est représenté ci-après :

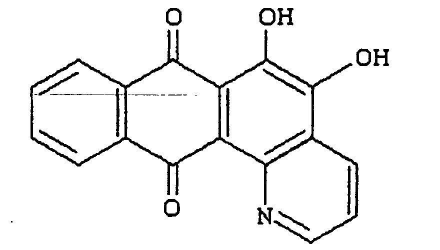

Les réactions de substitution et de cyclisation permettent d'obtenir un large éventail d'Alizarines modifiées ayant différentes propriétés.

L'état de la technique montre également que des applications biologiques et biomédicales existent déjà. En raison de la promptitude avec laquelle réagissent les Hydroxyanthraquinones en présence de chélates, celles-ci ont trouvées une application préférentielle comme tests de détection de la présence ou de l'absence de métaux dans des échantillons biologiques.

Les Anthrarobines, également appelées Désoxyalizarine ou Anthracène-1,2,10-triol, qui sont des produits réduits des Alizarine, sont déjà bien connu de l'homme du métier. Cette réduction s'effectue par l'action d'Hydroxyde de zinc ou de solution ammoniacale, de Chlorure stanneux d'acide, etc. La formule générale de ce composé est la suivante :

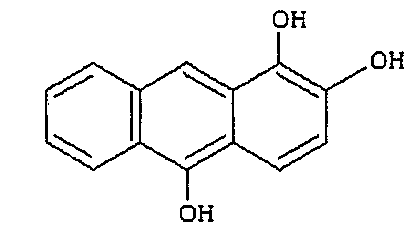

Toutefois, aucune information n'a, à ce jour, mis en évidence l'utilisation qui peut être faite des Alizarines, des dérivés Anthraquinoïdes ou des Anthrarobines comme substrats de détection d'une activité enzymatique. Ceci nécessite donc une synthèse de substrats où l'on fixe au moins une partie cible sur l'Alizarine. Ces substrats ont l'avantage de ne pas réagir en présence des ions métalliques, à partir du moment où l'hydrolyse en deux parties, marqueur et cible, n'a pas eu lieu. Pourtant la formation de chélates insolubles présente de substantiels avantages, qui sont les suivants :
- grande sensibilité, même à faible concentration, d'où peu de quantité de substrat nécessaire,
- couleur du produit de l'hydrolyse facilement adaptable aux besoins de l'application, de par la composition du milieu réactionnel et notamment les cations polyvalents (dont la définition sera donnée dans les points particuliers à la fin de la description) et/ou le pH utilisé(s),
- synthèse aisée et faible quantité de substrat nécessaire (due à la grande sensibilité évoquée plus haut), ce qui diminue les coûts de fabrication,
- diffusion de la coloration très faible, ce qui permet d'isoler et de différencier facilement les colonies, et
- faible inhibition car on utilise une faible quantité de substrat (due à la grande sensibilité évoquée plus haut).

Des dérivés d'Alizarine ont donc été synthétisés, principalement associés à des glycosides, par l'intermédiaire d'une méthode assez classique, dite de Koenigs-Knorr (Koenigs, W. and Knorr, E., Ber., 34, 957, 1901). En ce qui concerne les α-glycosides, leur synthèse est réalisée en modifiant la méthode Helferich (Helferich B. et *al.,* Ber., 66, 378 (1933) et Ber., 77, 194 (1944). D'autres dérivés sont associés à des esters à courtes chaînes d'acides gras, et des esters d'acides phosphorique ou sulfurique.

A ce jour, les substrats utilisés sont, par exemple le 5-Bromo-4-chloro-3-indolyl-β-D-galactoside, qui font ultérieurement l'objet d'une étude comparative avec un des substrats selon l'invention, l'Alizarine-β-D-galactoside.

Conformément à la présente invention, les substrats selon l'invention sont sensiblement plus efficaces que ceux décrit par l'état de la technique. Ainsi, ils détectent un plus grand nombre d'espèces et/ou souches de micro-organismes pour une même activité enzymatique étudiée.

Des substrats selon l'invention sont plus ou moins décrits dans d'autres documents.

Ainsi, un article de Masawaki. Teruyuki et al., « Selective solvent extraction of ruberythric acid from madder roots and subsequent hydrolysis with β-glucosidase », J. Ferment. Bioeng. (1996), 81(6), 567 569, concerne une extraction d 'Anthraquinones de racines de garance par utilisation d'un solvant sélectif, en vue de leur utilisation comme colorant. L'objectif est d'extraire entre autres les Alizarines parmi les Anthraquinones associées à des sucres, dont l'Alizarine-2-o-primeveroside. Pour cela, ils hydrolysent l'Alizarine-2-o primeveroside par l'intermédiaire de la β-glucosidase.

Un autre article provient de Van der Plas, Linus H. W. et al., « Anthraquinone glycosylation and hydrolysis in Morinda citrifolia cell suspensions. Regulation and function », J. Plant. Physiol., (1998), 152(2/3), 235-241, propose une explication biologique à la présence d'un sucre le Primeveroside glycosylé dans les cellules de plantes (voir page 240 colonne 1 3^{ème} alinéa). Cette source est due à l'hydrolyse de certaines Anthraquinones.

Un dernier article émane de Mateju. J al.. « Microbial glucosidation if dihydroxyanthraquinones. General properties of glucosidation system », Folia Microbiol. (Prague) (1974), 19(4), 307-316 qui a trait à l'activité « glucosidation » de l'espèce mutante Streptomyces aureofaciens B96.

Néanmoins, ceux-ci n'ont qu'un rapport lointain avec l'invention de la Demanderesse. Certes, tous les trois parlent de substrats à base d'Alizarine (ou autres Anthraquinones), mais ces substrats sont limités dans leur diversité (peu de molécules évoquées) et sont tous obtenus par biosynthèse (substrats associant un Primeveroside et produits par des plantes pour les deux premiers articles, substrats associant divers Glucosides et produits par la bactérie *Streptomyces griseus* pour le troisième article. Par ailleurs, ces substrats ne servent pas à générer des tests de diagnostic reposant sur la détection d'enzymes.

A cet effet, la présente invention concerne un substrat chromogène permettant de détecter la présence d'une activité enzymatique, de formule générale : 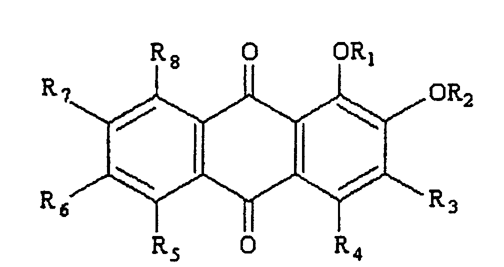 dans laquelle :
- R₁ est une partie cible ou H et R₂ est une partie cible ou H, au moins l'un des R₁ et R₂ étant une partie cible,
- R₃ est H, SO₃H, Cl, Br, F, I, NO₂, NH₂, NR₉R₁₀, Acylamino, Aminoaryl ou Aminoacylamino du type NHCOX, avec X égal à Alkyl, Aryl et Aralkyl ou un résidu d'acide α-aminé, tel que l'Alanine,
- R₄ est H, SO₃H, Cl, Br, F, I, NO₂, NH₂, NR₉R₁₀, OH, Acylamino, Aminoaryl ou Aminoacylamino du type NHCOX, avec X égal à Alkyl, Aryl, Aralkyl ou un résidu d'acide α-aminé, tel que l'Alanine,
- selon une variante, R₃ et R₄ sont reliés l'un à l'autre pour former un cycle à au moins cinq côtés, et préférentiellement à six côtés,
- R₅, R₆, R₇ et R₈ sont chacun constitués d'un des atomes ou groupements d'atomes suivants : H, halogène, particulièrement Cl ou Br, OH, SO₃H, Alkyl ou Alkoxy, et
- R₉ et R₁₀ sont constitués indépendamment de Méthyl, Alkyl, Aryl, Aralkyl ou constituent l'un, R₉ ou R₁₀, un cycle (Pipéridine, Pyrrolidine, Morpholine, etc.) et l'autre, R₁₀ ou R₉, un atome d'Hydrogène.

Dans un cas particulier, les cétones du cycle central sont réduites sous forme de groupements Hydroxyde, dans lesquels au moins un des atomes d'hydrogène est éventuellement remplacé par un groupement Méthyl, Alkyl, Aryl, Aralkyl.

La présente invention concerne également un substrat chromogène permettant de détecter la présence d'une activité enzymatique, de formule générale : 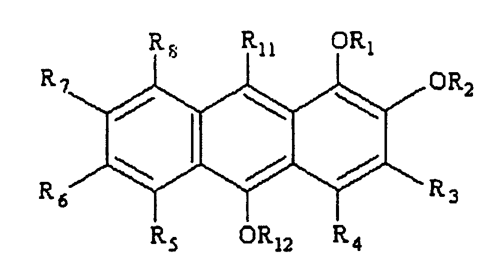 dans laquelle :
- R₁ est une partie cible ou H et R₂ est une partie cible ou H, au moins l'un des R₁ et R₂ étant une partie cible,
- R₃ est H, SO₃H, Cl, Br, F, I, NO₂, NH₂, NR₉R₁₀, Acylamino, Aminoaryl ou Aminoacylamino du type NHCOX, avec X égal à Alkyl, Aryl, Aralkyl ou un résidu d'acide α-aminé, tel que l'Alanine,
- R₄ est H, SO₃H, Cl, Br, F, I, NO₂, NH₂, NR₉R₁₀, OH, Acylamino, Aminoaryl ou Aminoacylamino du type NHCOX, avec X égal à Alkyl, Aryl, Aralkyl ou un résidu d'acide α-aminé, tel que l'Alanine,
- selon une variante, R₃ et R₄ sont reliés l'un à l'autre pour former un cycle à au moins cinq côtés, et préférentiellement à six côtés,
- R₅, R₆, R₇ et R₈ sont chacun constitués d'un des atomes ou groupements d'atomes suivants : H, Halogène, particulièrement Cl ou Br, OH, SO₃H, Alkyl ou Alkoxy,
- R₉ et R₁₀ sont constitués indépendamment de Méthyl, Alkyl, Aryl, Aralkyl ou constituent l'un, R₉ ou R₁₀, un cycle (Pipéridine, Pyrrolidine, Morpholine, etc.) et l'autre, R₁₀ ou R₉, un atome d'Hydrogène,
- R₁₁ est constitué d'un des atomes ou groupements d'atomes suivants : H, SO₃H, Cl, Br, F, I, NO₂, NH₂, NR₉R₁₀, Alkyl, Aryl, Aralkyl, Acylamino, Aminoaryl ou Aminoacylamino du type NHCOX, avec X égal à Alkyl, Aryl, Aralkyl ou un résidu d'acide α-aminé, et
- R₁₂ est constitué de H, Méthyl, Alkyl, Aryl, Aralkyl.

Dans un cas particulier où l'un des substrats ci-dessus est hydrolysé, la partie marqueur est constituée d'Alizarine, qui comporte deux groupements Hydroxyles en positions 1 et 2. Cette molécule a pour autre nom le 1,2-Dihydroxyanthraquinone-β-D-galactoside, dont la formule générale, associée à une partie cible constituée par du galactose, est la suivante :

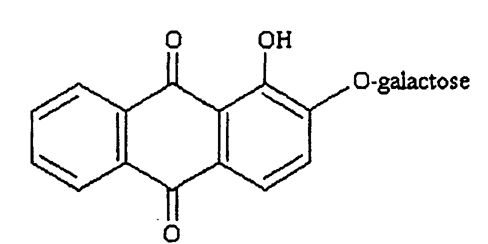

Le produit de son hydrolyse par une β-galactosidase forme un chélate en présence d'un sel de fer qui est le suivant :

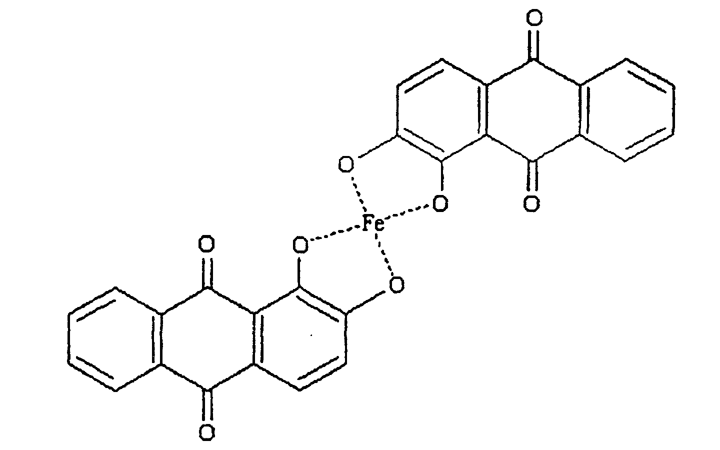

Dans tous les cas de figure ci-dessus développés et préférentiellement, R₁ est H et R₂ est la partie cible.

Plus précisément, la partie cible est constituée par l'une des molécules suivantes :
- un Glycoside, constitué par des unités mono-, di- et/ou polysaccharides, liés en α ou β à la fonction Hydroxyle,
- un acide α-aminé ou un peptide,
- un acide organique, tel que -O-CO-(CH₂)ₙ-CH₃, avec n compris entre 0 et 20, ou
- un Sulfate, un Phosphate, un Pyrosulfate, un Pyrophosphate ou un Phosphodiester.

Dans un cas particulier, R₃ et R₄ sont constitués et reliés l'un à l'autre par une chaîne en C₃N, substituée ou non, avec préférentiellement N adjacent de R₃ ou de R₄, afin de former un cycle à six côtés.

Selon un premier mode d'utilisation d'au moins un substrat, tel que décrit ci-dessus pour permettre de détecter la présence d'une activité enzymatique, il consiste :
- à mettre au moins un substrat, dont la partie cible est en relation avec l'activité enzymatique à détecter, en présence d'un échantillon suspecté de contenir au moins un micro-organisme ayant une telle activité enzymatique, et d'au moins un type de cation, et
- à observer la formation d'un chélate insoluble et coloré.

Préférentiellement, le substrat est mis en présence d'au moins un type de cation approprié par rapport à la partie marqueur libérée par l'activité enzymatique.

Toujours préférentiellement, un type de cation, qui peut être utilisé pour former un chélate insoluble, est constitué par : Fe²⁺, Al³⁺, Mn²⁺, Sn²⁺ ou Cu²⁺.

Dans le cas d'une utilisation d'au moins deux substrats, tels que précédemment décrits, pour permettre de détecter la présence d'au moins deux activités enzymatiques différentes, l'utilisation consiste :
- à mettre au moins deux substrats, dont les parties cibles sont en relation avec les au moins deux activités enzymatiques à détecter, en présence d'un échantillon suspecté de contenir au moins un micro-organisme ayant de telles activités enzymatiques, et d'au moins un type de cation, et
- à observer la formation d'au moins deux colorations différentes ou d'une tierce coloration.

Selon ce dernier cas, les substrats sont mis en présence d'au moins un type de cation, préférentiellement d'un seul type de cation, approprié par rapport aux parties marqueurs libérées par les activités enzymatiques.

Dans tous les cas de figure, l'utilisation d'au moins un substrat, tel que décrit plus haut, pour permettre de détecter la présence d'une activité enzymatique, ou cette utilisation en combinaison avec une utilisation déjà décrite précédemment, consiste :
- à mettre au moins un substrat, dont la partie cible est en relation avec l'activité enzymatique à détecter, en présence d'un échantillon suspecté de contenir au moins un micro-organisme ayant une telle activité enzymatique, dans un milieu réactionnel ayant un pH approprié, et
- à observer la formation d'au moins une coloration.

Préférentiellement, dès que l'on utilise au moins deux substrats, il y a utilisation d'un seul type de cation, approprié par rapport aux parties marqueurs libérées par les activités enzymatiques.

Selon un mode d'utilisation préférentiel, les utilisations ci-dessus incluent une étape intermédiaire consistant à laisser pousser le ou les micro-organismes sur un milieu de culture contenant le ou les substrats.

Si l'on souhaite détecter une activité enzymatique glycosidase, on utilise, entre autres, comme partie cible :
- Glucose,
- Galactose,
- Mannose,
- Xylose,
- Acide glucuronique, ou
- N-acétylglucosamine.

Si l'on souhaite détecter une activité enzymatique phosphatase, on utilise, entre autres, comme partie cible, l'Acide phosphorique ou un dérivé substitué.

Si l'on souhaite détecter une activité enzymatique sulfatase, on utilise, entre autres, comme partie cible, l'Acide sulfurique ou un dérivé substitué.

Si l'on souhaite détecter une activité enzymatique lipase ou phospholipase ou estérase, on utilise, entre autres, comme partie cible :
- un acide gras, saturé ou non, ou un dérivé substitué,
- l'Acide acétique ou un dérivé substitué,
- l'Acide butyrique ou un dérivé substitué,
- l'Acide octanoïque ou un dérivé substitué, ou
- un groupement phosphate estérifié, tel que l'inositol-1-phosphate.

L'invention concerne également une composition permettant la détection d'au moins une souche et/ou une espèce de micro-organismes qui comprend au moins un substrat, tel que défini ci-dessus, et un milieu de culture.

Dans le cas où la composition comporte au moins deux substrats, les produits, après réaction, sont de couleurs différentes permettant de différencier différentes activités enzymatiques révélées d'au moins une souche et/ou une espèce de micro-organismes.

Préférentiellement, la composition est constituée par un milieu de culture liquide, gélifié ou solide (par exemple sec pouvant être repris dans un liquide idoine).

Toujours préférentiellement, la concentration du substrat est comprise entre 10 et 500 mg/l, préférentiellement entre 30 et 150 mg/l, et encore plus préférentiellement est de 50 mg/l.

La présente invention concerne donc un nouveau substrat faiblement coloré initialement qui prend une coloration particulière en présence, d'une part, d'un micro-organisme ou d'une enzyme dont on veut détecter la présence, d'autre part et éventuellement, d'un cation. L'invention concerne également les utilisations qui peuvent être faites de ce substrat, ainsi qu'une composition contenant ce substrat.

La présence de cations est particulièrement intéressante, néanmoins il est possible de s'en passer ; dans ce cas, on peut préférentiellement utiliser une composition ayant un pH alcalin. Il est également possible de cumuler les deux conditions, c'est-à-dire la présence de cations et un pH alcalin.

Lorsque le cation utilisé varie, les colonies de micro-organismes testées donnent des colorations différentes. Ces cations, tels que Fer, Manganèse, Etain et Aluminium, sont utilisés à très faibles concentrations afin d'éviter ou de minimiser l'inhibition due à des ions libres présents dans l'échantillon testé.

Cependant des concentrations plus élevées de certains ions métalliques donne une inhibition sélective qui peut être utilisée pour sélectionner des espèces microbiennes particulières, ce qui constitue un avantage supplémentaire.

### SYNTHESE DES SUBSTRATS :

### 1°) Synthèse de l'Alizarine-2-β-D-glucoside:

Selon Robertson *et al.* (J. Chem. Soc. (1930), 1136 et (1933), 1167), l'Alizarine est glycosylée spécifiquement au niveau du groupement Hydroxyle en position 2, même s'il est possible d'effectuer cette fixation sur la position 1. Néanmoins, la couplage en position 2 est plus aisée.

Ce substrat est préparé en utilisant la méthode décrite par Robertson (1933) mais modifiée. Un échantillon de 6 g d'Alizarine est suspendu dans 70 ml d'Acétone et mélangé avec 70 ml d'Hydroxyde de Potassium à 0,28 mol/l afin de former un sel. A ceci est associé un mélange de 40 ml contenant dans un rapport 1:1 de l'Ether et de l'Acétone, contenant 6,6 g d'Acéto-bromo-glucose. On agite environ 14 heures. On ajoute ensuite 7 ml d'Hydroxyde de potassium à 1,25 mol/l, suivi de l'ajout de 15 ml à 0,6 mol/l Acéto-bromo-glucose dans l'Acétone. Le prémélange est encore agité une dizaine d'heures. L'Ether et l'Acétone sont retirés sous pression réduite et le pH est ramené à environ 5,5 par addition d'Acide acétique glacé. Le mélange et l'Alizarine n'ayant pas réagi sont filtrés puis lavés à l'eau, pour ensuite être séchés toute la nuit à 50°C.

On obtient ainsi un solide jaune qui est mis en suspension dans 70 ml d'Acide acétique glacé et reflué pendant 5 minutes. Ceci permet de refroidir puis de filtrer et enfin de laver à l'Acide acétique. Le filtrat est ensuite mis de côté pendant une heure et séparé de l'Alizarine restante. Cette procédure est répétée à 10°C afin d'obtenir un filtrat jaune foncé.

Ce que l'on obtient est séché et dissous dans 200 ml de Méthane dichlorique et on ajoute ensuite 2 ml de Triéthylamine. Un Oxyde d'Aluminium est prémélangé dans la solution jusqu'à ce qu'un échantillon test montre qu'il ne reste pas d'Alizarine par chromatographie sur couche mince (CCM). L'Oxyde d'Aluminium est retiré et la solution restante est évaporée par mise en rotation afin de produire un solide jaune. Ce solide est recristallisé à partir d'Ethanol chaud en présence de quelques gouttes d'Acide acétique pour obtenir 2,02 g de Tétraacétyl-glucoside d'Alizarine.

Une quantité de 1,1 g du Tétraacétyl-gucoside d'Alizarine est remis en suspension dans 60 ml d'Ethanol, on y ajoute 30 ml d'Hydroxyde de Sodium aqueux à 0,125 mol/l pour produire une solution rouge. Cet état est maintenu pendant 10 minutes à 65°C puis refroidi à 0°C. Le sel de Sodium de l'Alizarine glycosylée rouge est ensuite retiré par filtration à vide, lavé avec l'Ether puis séché. Ce procédé permet d'obtenir 0,9 g de sel de Sodium d'Alizarine-2-β-D-glucoside. On peut purifier ce produit selon des techniques connue de l'homme du métier pour obtenir de l'Alizarine-2-β-D-glucoside.

### 2°) Synthèse de l'Alizarine-2-β-D-galactoside :

Ce substrat est également préparé en utilisant la méthode décrite par Robertson (1933) mais modifiée. Un échantillon de 6 g d'Alizarine est suspendu dans 70 ml d'Acétone et mélangé avec 70 ml d'Hydroxyde de Potassium à 0,28 mol/l afin de former un sel. A ceci est associé un mélange de 40 ml contenant dans un rapport 1:1 de l'Ether et de l'Acétone, contenant 6,6 g d'Acéto-bromo-galactose. On agite environ 14 heures. On ajoute ensuite 7 ml d'Hydroxyde de Potassium à 1,25 mol/l, suivi de l'ajout de 15 ml à 0,6 mol/l d'Acéto-bromo-galactoside dans l'Acétone. Le prémélange est encore agité une dizaine d'heures. L'Ether et l'Acétone sont retirés sous pression réduite et le pH est ramené à environ 5,5 par addition d'Acide acétique glacé. Le mélange et l'Alizarine n'ayant pas réagi sont filtrés puis lavés à l'eau, pour ensuite être séchés toute la nuit à 50°C.

Ce que l'on obtient est dissous dans 200 ml de Méthane dichlorique et on ajoute ensuite 2 ml de Triéthylamine. Un Oxyde d'Aluminium est ajouté dans la solution jusqu'à ce qu'un échantillon test montre qu'il ne reste pas d'Alizarine par CCM. L'Oxyde d'Aluminium est retiré et la solution restante est évaporée par mise en rotation afin de produire un solide jaune. Ce solide est recristallisé à partir d'Ethanol chaud en présence de quelques gouttes d'Acide acétique pour obtenir 1,96 g de Tétraacétyl-galactoside d'Alizarine.

Une quantité de 1,1 g de Tétraacétyl-galactoside d'Alizarine est remis en suspension dans 60 ml d'Ethanol, on y ajoute 30 ml d'Hydroxyde de Sodium aqueux à 0,125 mol/l pour produire une solution rouge. Cet état est maintenu pendant 10 minutes à 65°C puis refroidi à 0°C. Le sel de Sodium de l'Alizarine glycosylée rouge est ensuite retiré par filtration à vide, lavé avec l'Ether puis séché. Ce procédé permet d'obtenir 0,86 g de sel de Sodium d'Alizarine-2-β-D-galactoside sous forme de poudre rouge microcristalline.

### 3°) Synthèse de l'Alizarine-2-acétate :

Ce substrat est préparé par une technique bien connue de l'homme du métier. Deux grammes d'Alizarine sont dissous dans 5 ml de Pyridine et traités par un mélange de 2,5 ml d'Anhydride acétique et 5 ml de Pyridine. Après 16 heures à température ambiante, la solution jaune est versée dans 100 ml d'Acide chlorhydrique contenant de la glace. Le précipité d'Acétate est retiré par filtration aspirante et lavé à l'eau. La recristallisation d'Acétone aqueux permet d'obtenir 1,4 g de Alizarine-2-acétate sous la forme de cristaux jaunes.

### 4°) Synthèse de l'Alizarine-2-sulfate :

Ce substrat est préparé par chauffage de 2,4 g d'Alizarine, soit 10 mmol, dans 10 ml de Pyridine contenant 4 g d'un complexe Pyridine-trioxyde de sulfure. Après 2 heures à 60°C, la Pyridine est reprise sous une pression réduite. L'Ester de sulfate est cristallisé, comme le sel de Potassium, par ajout méticuleux d'une Potasse méthanolique à un pH de 9. Le sel de Potassium se forme graduellement et est repris par filtration aspirante et lavage par un Ether pour donner 1,2 g d'une poudre microcristalline blanche, l'Alizarine-2-sulfate.

### 5°) Synthèse de l'Alizarine-1-galactoside:

Ce substrat est préparé à partir de l'Alizarine-2-acétate, décrit précédemment au chapitre 3°). Ces 2,82 g d'ester, soit 10 mmol, est mélangé en présence de 75 ml de Méthane dichlorique et on ajoute au mélange 2 à 3 ml de 2,4,6-Collidine ou de 2,6-Lutidine afin d'obtenir une solution d'un pourpre profond. Après une heure, du carbonate d'argent, préparé selon Wolfrom et Lineback, « Methods in Carbohydrate Chemistry 2 » (1963), 342-43, suivi de l'ajout de 5 g, soit 12,5 mmol, d'Acéto-bromo-galactose. La réaction se poursuit à température ambiante, c'est-à-dire 10 à 15°C, pendant deux jours avec agitation dans un récipient. Une chromatographie sur couche mince montre une conversion progressive en Tétra-acétyl-galactoside, qui migre rapidement sur la chromatographie. La suspension est filtré un lit de Silice ou de Diatomite grossière et l'adjuvant de filtrage est laver par du Dichloro-méthane en quantité nécessaire, soit approximativement 100 ml. La solution de composé de Dichloro-méthane est ensuite lavé en présence de 0,2 M d'acide chlorhydrique (3 x 100 ml) à l'eau (x 2). Après séchage (MgSO₄), l'extrait brun clair est évaporé sous pression réduite puis dissous à nouveau dans du méthanol. Une chromatographie sur couche mince (Acétate d'éthyl/Toluène 3 :1) indique la présence d'un composant migrant rapidement et donnant une réaction positive aux ultraviolets et à l'acide sulfurique. Le Galactoside protégé est ensuite désacétylé, comme déjà décrit en utilisant du Méthoxide de Sodium dans du Méthanol pour donner 1,32 g d'Alizarine-1-galactoside.

### 6°) Synthèse de l'Alizarine-1-phosphate-2-octanoate :

Ce substrat est préparé en faisant réagir 2,4g, soit 10 mmol, dans 100 ml de Dichloro-méthane et 3 ml de Triéthylamine. En plus, 1,62 g de Chlorure d'octanoyl, soit 10 mmol, est ajouté lentement pendant 30 minutes à température ambiante à cette solution en cours de mélange. L'Octanoate est purifié en le traitant avec de l'Alumine, comme cela a déjà été décrit, et isolé en retirant le solvant et cristallisation dans le Méthanol. Après refroidissement à -12°C, une quantité de 1,84 g d'Octanoate, soit 5 mmol, dans 30 ml de l'Acétonitrile sec, est traitée successivement avec :
- 3,6 g de Tétrachlorure de carbone,
- 1,6 g de Di-isopropyléthylamine, et
- 80 mg de 4-diméthylaminopyridine.
Après approximativement 2 minutes à basses températures, une solution contenant 2,2 g de Dibenzyl-phosphite est ajoutée dans 8 ml d'Acétonitrile, ce qui éviteune montée en température significative. Après une heure, le mélange est exploité comme décrit par Silverberg L. J., Dillon J. L. et Vermeshetti P., Tet. Lett., 37 N°6 (1996), et l'Ester de dibenzyl-phosphoryl est détruit à l'Hydrogène en utilisant 30 ml d'Ethyl d'acétate comme solvant, avec 0,4 g d'un catalyseur Palladium/Carbone (10% w/w). L'Ester de phosphate est ensuite isolé, comme le sel de Potassium, après retrait d'Ethyl d'acétate par remise en solution dans le Méthanol et ajout précautionneux d'une solution de Carbonate de potassium dans du Méthanol aqueux à pH 8. Le précipité de sel de Potassium d'Alizarine-1-acide phosphorique-2-octanoate est recueilli, lavé au Méthanol, et les 2,05 g d'Ester sont séchés sous vide et utilisés sans autre purification.

### 7°) Synthèse de la Déoxyalizarine :

Ce type de substrat est préparé selon la synthèse exposée dans Liebermann - Ber., 21 444 (1888). Pour la synthèse des dérivés 9- et 10-O-Méthyl des Anthraquinones réduites, l'ensemble des 1,2-diol est protégé préalablement à la méthylation par un processus approprié, connu de l'homme du métier, c'est-à-dire par complexion avec du borate, comme décrit dans Scheline - Acta. Chem. Scand. 20 1182 (1966) ou par l'utilisation d'Acétaldéhyde di-méthyl acétate (protection par l'Ethylidène).

### APPLICATIONS

De nombreuses applications sont possibles :
1. Détection et localisation d'espèces microbiennes spécifiques dans un milieu solide ou sur membrane.
2. Détection d'activités enzymatiques en solution à partir d'extraits tissulaires ou cellulaires, ou à partir de suspension de cellules d'eucaryotes ou de procaryotes.
3. Identification d'organismes en fonction de la présence d'activités enzymatiques.
4. Visualisation et localisation d'une réaction spécifique entre antigène et anticorps, telle que la technique de l'ELISA. On peut par exemple utiliser de l'Alizarine-β-galactoside ou de l'Alizarine-phosphate pour des technologies destinées à révéler les activités β-galactosidase ou phosphatase alcaline comme enzymes indicatrices. Dans ce cas, les substrats enzymatiques sont utilisés tout d'abord dans des réactions de détection où une activité enzymatique (notamment la phosphatase alcaline ou la β-galactosidase) est mise en jeu, comme les réactions de détection d'anticorps ou d'antigène avec un format type ELISA, par exemple « Les immunodosages de la théorie à la pratique » coordonné par Y. Barbier, aux éditions de l'ACOMEN, Lyon, pages 109 à 133 (1988) ; ou encore dans la détection d'acides nucléiques, par exemple « DNA probes » 2^{ème} édition, Keller G.H., Manak, M.M., Stockton press, sections 5 à 9 (1993).
5. Techniques de biologie moléculaire où il y a lieu de démontrer la présence d'un gène par exemple de la β-galactosidase et son utilisation « Molecular cloning a laboratory manual » 2^{ème} édition, Sambrook, Fritsch, Maniatis, Cold Spring Harbor Laboratory Press, sections 16.56 et section 1.85 (1989).
6. Techniques histochimique, cytochimique ou à cytométrie de flux. L'utilisation de tels substrats en relation avec des activités enzymatiques spécifiques a des applications dans le diagnostic médical et dans d'autres domaines, comme l'analyse de l'eau, de l'environnement, des aliments, etc.
7. Révélation d'enzymes sur des profils de gels polyacrylamides ou autres matériaux utilisés pour effectuer des électrophorèses ou d'autres méthodes de séparation.

### EXEMPLES

### Exemple 1 : Influence de la concentration de l'Alizarine-2-β-D-galactoside sur la révélation de l'activité β-galactosidase de micro-organismes sur milieu gélifié,

Dans le milieu ci-après, base Columbia à une concentration de 46,37 g/l, a été ajouté soit du Alizarine-2-β-D-galactoside à 0,01 g/l, 0,03 g/l, 0,05 g/l et 0,08 g/l en présence de Citrate de Fer Ammoniacal à 0,05 g/l, soit du 6-Chloro-3-indolyl-β-D-galactoside à 0,2 g/l sans Citrate de Fer Ammoniacal. Ces quatre milieux ont été répartis en boîte de Pétri à raison de 20 ml de milieu par boîte. Des micro-organismes ont été ensemencés sur ce milieu par isolement en trois cadrans à partir d'une suspension à 0,5 McFarland. Les boîtes ont été incubées à 37 °C pendant 48 heures. Les colonies formées ont été examinées visuellement après 18, 24 et 48 heures d'incubation. La coloration de ces colonies ainsi que l'intensité de cette coloration ont été notées. Les résultats sont présentés dans le tableau 1 ci-dessous :

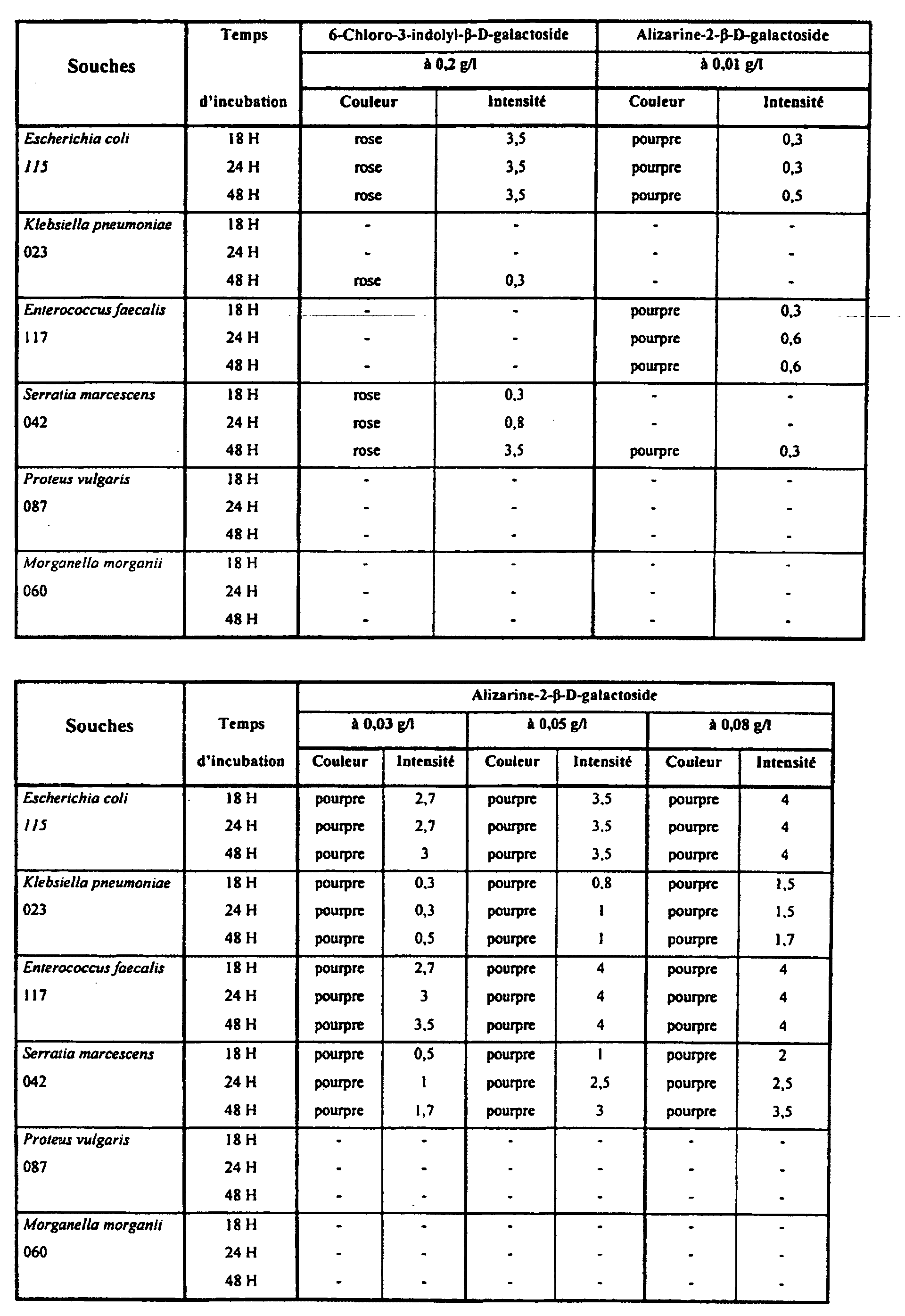

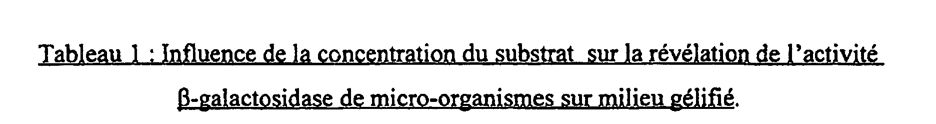

Le symbole « - » signifie, dans ce tableau 1, une absence de coloration. Les souches ainsi testées, n'ayant que des résultats négatifs, font office de contrôle négatif.

D'après ce tableau 1, il est possible de remarquer que les intensités obtenues avec le Alizarine-2-β-D-galactoside dès 0,03 g/l sont quasiment équivalentes à celles observées avec le 6-Chloro-3-indolyl-β-D-galactoside, dont la concentration est environ sept fois supérieure. La concentration en Alizarine-2-β-D-galactoside permettant d'obtenir des intensités de coloration intéressantes est comprise entre 0,03 g/l et 0,08 g/l préférentiellement 0,05 g/l.
Les substrats à base d'Alizarine sont donc plus sensibles que les substrats à base d'lndoxyl.

### Exemple 2 : Révélation de l'activité β-galactosidase de micro-organismes sur milieu gélifié - Utilisation du Alizarine-2-β-D-galactoside.

Un milieu gélifié comprenant du Alizarine-2-β-D-galactoside est préparé comme suit : 46,37 g d'agar Columbia sont additionnés à 1 litre d'eau distillée avec 50 mg de Alizarine-2-β-D-galactoside, 500 mg de Citrate de Fer Ammoniacal et 30 mg d'Isopropyl-β-D-thiogalactoside pour faciliter l'induction de l'activité β-galactosidase. L'agar est stérilisé par autoclavage à 116°C pendant 10 min. Le milieu est refroidi lentement à 55°C avant d'être réparti dans des boîtes de Pétri de 20 ml. Ce milieu est comparé à un milieu solide préparé de la même façon et contenant 46,37 g d'agar Columbia avec 80 mg de 5-Bromo-4-chloro-3-indolyl-β-D-galactoside et 30 mg d'Isopropyl-β-D-thiogalactoside.

Trois cent soixante-sept souches (367) différentes ont été collectées à partir d'échantillons cliniques et environnementaux et identifiés par la galerie API (marque déposée) 20E (BioMérieux, France) comme méthode de référence. Les souches sont cultivées sur un milieu Columbia agar à 37°C pendant 24 heures puis un inoculum d'environ 10⁸ organismes/ml (équivalent à un standard McFarland de 0,5) est réalisé pour chaque souche. En utilisant un inoculateur Denley, 1 microlitre de chaque suspension est inoculée sur une boite de chacun des milieux : le milieu contenant du Alizarine-2-β-D-galactoside préparé ci-dessus et le milieu contenant le 5-Bromo-4-chloro-3-indolyl-β-D-galactoside. Toutes les boîtes sont incubées à 37°C pendant 18 heures.

Après incubation, les colonies formées sont examinées visuellement. Sur le milieu contenant le Alizarine-2-β-D-galactoside les colonies présentant une activité β-galactosidase sont de couleur Pourpre et sur le milieu contenant le 5-Bromo-4-chloro-3-indolyl-β-D-galactoside, elles sont de couleur Turquoise. Toute souche présentant une de ces deux couleurs est considérée comme positive pour l'activité β-galactosidase avec le substrat correspondant. Les résultats sont présentés dans le tableau 2 ci-après.

**Tableau 2 :**

| Révélation de l'activité β-galactosidase de micro-organismes sur milieu gélifié contenant du Alizarine-2-β-D-galactoside | | | |
|---|---|---|---|
| **Espèces** | **Nombre de souches testées pour l'espèce** | **Alizarine-2-β- D-galactoside** | **5-Bromo-4-chloro-3-indolyl-2-β- D-galactoside** |
| *Acinetobacter spp.* | 53 | 0 | 0 |
| *Aeromonas caviae* | 7 | 86 | 86 |
| *Aeromonas hydrophila* | 3 | 100 | 100 |
| *Citrobacter diversus* | 9 | 89 | 89 |
| *Citrobacter freundii* | 16 | 100 | 100 |
| *Enterobacter aerogenes* | 9 | 100 | 100 |
| *Enterobacter agglomerans* | 1 | 100 | 100 |
| *Enterobacter cloacae* | 21 | 100 | 100 |
| *Escherichia coli* | 41 | 95 | 95 |
| *Escherichia hermannii* | 1 | 100 | 100 |
| *Hafnia alvei* | 10 | 80 | 80 |
| *Klebsiella oxytoca* | 13 | 100 | 100 |
| *Klebsiella ozaenae* | 3 | 100 | 67 |
| *Klebsiella pneumoniae* | 19 | 100 | 100 |
| *Morganella morganii* | 12 | 0 | 0 |
| *Proteus mirabilis* | 16 | 0 | 0 |
| *Proteus penneri* | 1 | 0 | 0 |
| *Proteus vulgaris* | 4 | 0 | 0 |
| *Providencia alcalifaciens* | 3 | 0 | 0 |
| *Providencia rettgeri* | 3 | 0 | 0 |
| *Providencia stuartii* | 10 | 0 | 0 |
| *Salmonella spp.* | 64 | 0 | 0 |
| *Serratia odorifera* | 1 | 100 | 100 |
| *Serratia spp.* | 14 | 86 | 79 |
| *Shigella boydii* | 1 | 0 | 0 |
| *Shigella dysenteriae* | 2 | 0 | 0 |
| *Shigella flexneri* | 2 | 0 | 0 |
| *Shigella sonnei* | 10 | 100 | 100 |
| *Vibrio cholerae* | 1 | 100 | 100 |
| *Yersinia enterocolitica* | 14 | 64 | 29 |
| *Yersinia pseudotuberculosis* | 3 | 67 | 0 |

Les chiffres présents dans les colonnes associées à l'Alizarine-2-β-D-galactoside et 5-Bromo-4-chloro-3-indolyl-β-D-galactoside correspondent aux pourcentages de souches positives. La grande majorité des souches, c'est-à-dire 96,5 %, a une réactivité identique, soit positive soit négative, pour les deux substrats. Huit souches n'hydrolysent que le Alizarine-2-β-D-galactoside. Or ces huit souches ont une activité β-galactosidase du fait de la détection d'une fluorescence en présence du substrat 4-Méthylumbelliféryl-β-D-galactoside. Ce tableau montre donc une bonne efficacité du substrat comme indicateur de l'activité β-galactosidase avec une sensibilité supérieure à celle observée avec le substrat de référence le 5-Bromo-4-chloro-3-indolyl--β-D-galactoside. Ces substrats sont donc très sensibles et utilisables à faible concentration.

### Exemple 3 : Effet de différents sels de métaux sur la coloration de la partie marqueur.

Dans le milieu, base Columbia (46,37 g/l) et Alizarine-2-β-D-glucoside (50 mg/l), ont été ajoutés à la concentration de 50 mg/l soit du Chlorure de Manganèse, soit du Citrate de Fer Ammoniacal, soit du Chlorure d'Etain, soit du Sulfate d'Aluminium. Un milieu témoin sans sel de métaux a aussi été étudié. Ces différents milieux ont été répartis après passage à autoclave en boîtes de Pétri à raison de 20 ml de milieu par boîte. Des micro-organismes issus de la collection de la demanderesse ont été ensemencés sur chacun des milieux par isolement en trois cadrans à partir d'une suspension à 0,5 McFarland. Les boîtes ont été incubées à 37 °C pendant 48 heures. Les colonies formées ont été examinées visuellement après 24 et 48 heures d'incubation. La coloration de ces colonies ainsi que l'intensité de cette coloration ont été notées. Les résultats sont présentés dans le tableau 3 ci-dessous. Il convient de noter que les valeurs d'intensité sont données arbitrairement, elles n'ont pour but que de permettre de comparer les intensités d'une souches à l'autre. Ceci est également vrai pour les exemples qui suivent. De même, les souches testées issues de la collection de la demanderesse, comportent un numéro interne à cette collection. Cette numérotation interne est également utilisée dans certains des exemples qui seront exposés par la suite.

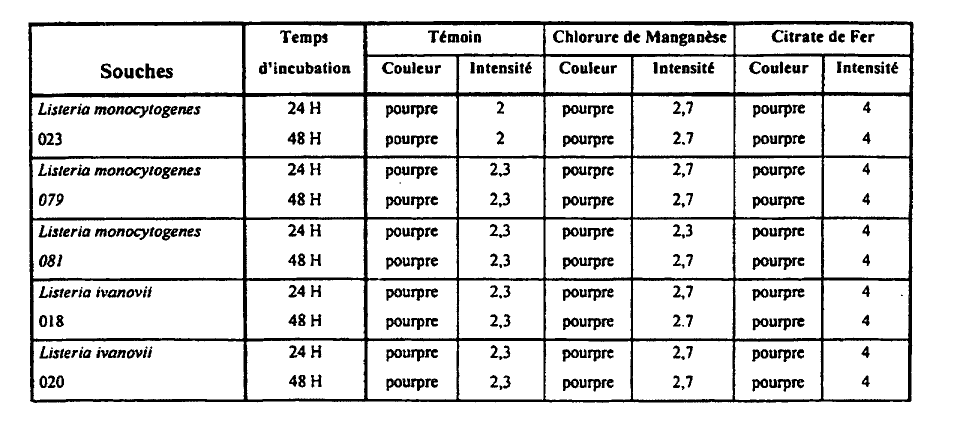

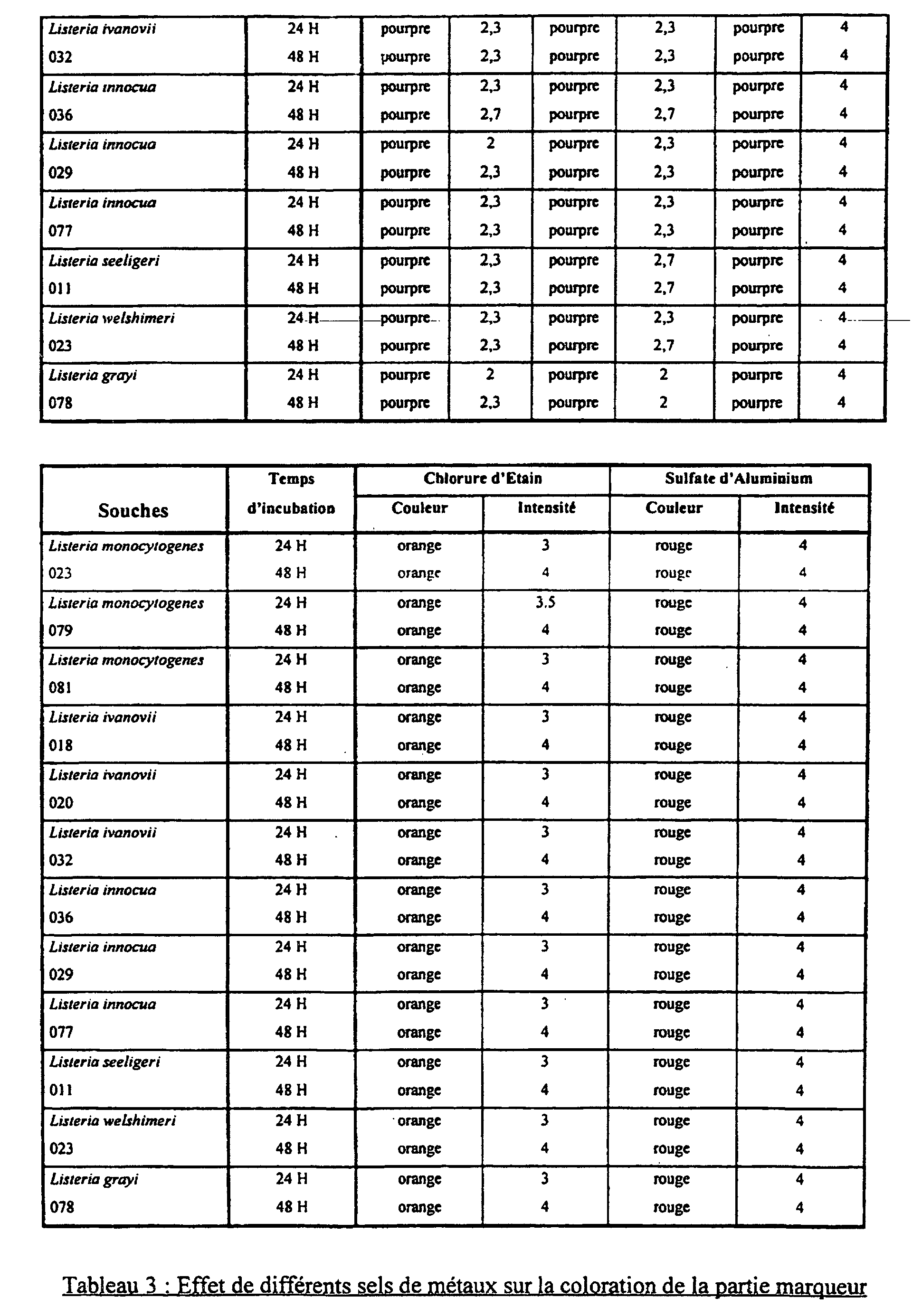

Suivant le sel de métal testé la couleur obtenue après hydrolyse du substrat est différente. Il est aussi possible d'observer une coloration même en absence de sels de métaux (milieu témoin). Cependant l'intensité de la coloration obtenue est inférieure à celle observée, par exemple avec le sel de Fer, sel qui ne modifie pas la couleur.

Ceci peut permettre d'adapter la couleur et son intensité en fonction des besoins (association avec d'autres substrats enzymatiques, indicateurs de pH).

### Exemple 4 : Influence du pH sur la révélation de la β-D-galactosidase en présence de Alizarine-2-β-D-galactoside.

Dans dix-huit tubes contenant 5 ml d'eau osmosée à pH 7 ont été ajoutés de l'Alizarine-2-β-D-galactoside à 0,05 g/l, du Citrate de Fer Ammoniacal à 0,05 g/l dans la moitié des tubes et 5 µl de β-galactosidase (EC 3.2.1.23 Sigma) dans chaque tube. Ces dix-huit tubes ont été incubés à 37°C pendant 4 H. Après incubation, la coloration obtenue est rose pâle pour les tubes ne contenant pas de Citrate de Fer Ammoniacal et rose plus intense pour les tubes contenant du Citrate de Fer Ammoniacal. Enfin, ces différents tubes ont été ajustés à différents pH (2 - 3 - 4 - 5 - 6 - 7 - 8 - 9 - 10) à 24°C. Les colorations obtenues après ajustement sont présentées dans le tableau 4 ci-après.

**Tableau 4 :**

| Influence du pH sur la révélation de la β-D-galactosidase en présence de 2-Alizarine-β-D-galactoside | | |
|---|---|---|
| **pH** | **Tubes ne contenant pas de Fer** | **Tubes contenant du Fer** |
| **pH 2** | Jaune | Jaune |
| **pH 3** | Jaune | Jaune |
| **pH 4** | Jaune | Jaune |
| **pH 5** | Jaune - Orangé | Jaune - Rose |
| **pH 6** | Rose - Orangé | Rose |
| **pH 7** | Rose - Orangé | Rose |
| **pH 8** | Rose | Pourpre |
| **pH 9** | Pourpre | Pourpre |
| **pH 10** | Mauve | Mauve |

La couleur varie en fonction du pH. En général, elle est jaune à pH acide et rose à pourpre à pH alcalin. Ceci peut permettre d'adapter la couleur en fonction des besoins ou de mettre en évidence plusieurs métabolismes (hydrolyse enzymatique et variation de pH). Ceci prouve également qu'il est possible d'utiliser une large palette de pH.

### Exemple 5 : Association d'un substrat à base d'Alizarine avec un autre substrat enzymatique dans un milieu gélosé - Détection d'au moins deux activités enzymatiques différentes

Le milieu suivant :
- base Columbia à une concentration de 46,37 g/l,
- Alizarine-2-13-D-glucoside à 0,05 g/l,
- Citrate de Fer Ammoniacal à 0,05 g/l,
- 5-Bromo-4-chloro-3-indolyl-β-D-galactoside à 0,05 g/l, et
- Isopropyl-β-D-thiogalactoside à 30 mg/l pour faciliter l'induction de l'activité β-galactosidase,
a été réparti en boîtes de Pétri à raison de 20 ml de milieu par boîte. Des micro-organismes issus de la collection de la demanderesse ont été ensemencés sur ce milieu par isolement en trois cadrans à partir d'une suspension à 0,5 McFarland. Les boîtes ont été incubées à 37 °C pendant 48 heures.

Les colonies formées ont été examinées visuellement après 24 et 48 heures d'incubation. La coloration de ces colonies ainsi que l'intensité de cette coloration ont été notées. Les résultats sont présentés dans le tableau 5 ci-dessous :

**Tableau 5 :**

| Association de deux substrats, l'Alizarine-2-β-D-glucoside et 5-Bromo-4-chloro-3-indolyl-β-D-galactoside | | | |
|---|---|---|---|
| **Souches** | **Temps d'Incubation** | **Alizarine-2-β-Glucoside et 5-Bromo-4-chloro-3-indolyl-β-D-galactoside** | |
| | | **Couleur** | **Intensité** |
| *Escherichia coli* | 24 H | Turquoise | 3.5 |
| 115 | 48 H | Turquoise | 4 |
| *Escherichia coli* | 24 H | Turquoise | 3.5 |
| 206 | 48 H | Turquoise | 4 |
| *Citrobacter freundii* | 24 H | Turquoise | 3.5 |
| 136 | 48 H | Turquoise | 3.5 |
| *Enterococcus faecalis* | 24 H | Pourpre | 4 |
| 117 | 48 H | Pourpre | 4 |
| *Enterococcus faecalis* | 24 H | Pourpre | 4 |
| 066 | 48 H | Pourpre | 4 |
| *Enterococcus faecium* | 24 H | Pourpre | 4 |
| 039 | 48 H | Pourpre | 4 |
| *Klebsiella pneumoniae* | 24 H | Bleu Mauve | 4 |
| 023 | 48 H | Bleu Mauve | 4 |
| *Enterobacter cloaecae* | 24 H | Bleu Mauve | 4 |
| 059 | 48 H | Bleu Mauve | 4 |
| *Citrobacter koseri* | 24 H | Bleu Mauve | 4 |
| 002 | 48 H | Bleu Mauve | 4 |
| *Morganella marganii* | 24 H | - | - |
| 035 | 48 H | - | - |
| *Pseudomonas aeruginosa* | 24 H | - | - |
| 054 | 48 H | - | - |
| *Proteu mirabilis* | 248 H | - | - |
| 154 | 48 H | - | - |

Le symbole « - » signifie, dans ce tableau 5, une absence de coloration. Les souches ainsi testées, n'ayant que des résultats négatifs, font office de contrôle négatif.

Il est possible grâce à l'association de ces deux substrats de détecter quatre groupes de micro-organismes différents :
- le premier, présentant une coloration Turquoise, correspond aux espèces ne possédant que l'activité β-galactosidase,
- le deuxième, présentant une coloration Pourpre, correspond aux espèces ne possédant que l'activité β-glucosidase,
- le troisième groupe présentant un mélange des deux colorations précédentes, c'est à dire variant du Bleu au Mauve correspond aux espèces possédant les deux activités enzymatiques détectées, et
- le quatrième groupe, incolore, correspond aux espèces ne possédant aucune des deux activités précédemment citées.

Il est donc possible avec ces substrats à base d'Alizarine d'associer d'autres substrats enzymatiques afin de différencier un ou plusieurs groupes de micro-organismes en fonction des activités biochimiques présentes chez ces micro-organismes.

### Exemple 6: Révélation de l'activité β-glucosidase de micro-organismes en milieu liquide - Utilisation du Alizarine-2-β-D-glucoside.

Dans une cupule de galerie type API (marque déposée, bioMérieux, France), 3 µl d'un mélange de Alizarine-2-β-D-glucoside à 0,12 g/l et de Citrate de Fer Ammoniacal à 0,05 g/l ont été déposés puis séchés. Une cupule témoin, contenant du 6-Chloro-3-indolyl-β-D-glucoside à la concentration finale de 0,4 g/l, a été réalisée. Ces deux cupules ont alors été inoculées avec 50 µl d'une base Columbia sans agar et 100 µl d'une suspension bactérienne à 2 McFarland. Après 4 et 24 heures d'incubation à 37°C, la coloration obtenue dans la cupule a été notée. Les résultats sont présentés dans le tableau 6 ci-dessous :

**Tableau 6 :**

| Révélation de l'activité β-glucosidase de micro-organismes en milieu liquide contenant du Alizarine-2-β-D-glucoside. | | | |
|---|---|---|---|
| **Souches** | **Temps d'incubation** | **Couleur observée avec Alizarine-2-β-D-glucoside** | **Couleur observée avec 6-Chloro-3-indolyl-β-D-glucoside** |
| *Listeria monocylogenes* | 4 H | Pourpre | Rose |
| 022 | 24 H | Pourpre | Rose |
| *Listeria ivanovii* | 4 H | Pourpre | Rose |
| 018 | 24 H | Pourpre | Rose |
| *Listeria innocua* | 4 H | Pourpre | Rose |
| 036 | 24 H | Pourpre | Rose |
| *Listeria seeligeri* | 4 H | Pourpre | - |
| 080 | 24 H | Pourpre | Rose |
| *Bacillus thuringiensis* | 4 H | - | - |
| 072 | 24 H | Pourpre | - |
| *Klebsiella pneumoniae* | 4 H | Pourpre | Rose |
| 023 | 24 H | Pourpre | Rose |
| *Staphylococcus aureus* | 4 H | - | - |
| 062 | 24 H | - | - |
| *Enterococcus faecium* | 4 H | Pourpre | Rose |
| 009 | 24 H | Pourpre | Rose |

Le symbole « - » signifie, dans ce tableau 6, une absence de coloration. Les souches ainsi testées, n'ayant que des résultats négatifs, font office de contrôle négatif.

Le substrat à base d'Alizarine permet de détecter une activité pour sept (7) espèces sur huit (8) testées, alors que le 6-Chloro-3-indolyl-β-D-glucoside ne permet de détecter une activité que pour six (6) de ces espèces. D'autre part, pour deux souches *(Bacillus thuringiensis* et *Listeria seeligeri)* l'activité est détectée plus précocement avec le substrat à base d'Alizarine. Ces substrats sont donc, d'une part, utilisables en milieu liquide et, d'autre part, plus sensibles que les substrats à base d'Indoxyl.

### Exemple 7 : Comparaison de l'Alizarine-1-β-D-glucoside et de l'Alizarine-2-β-D-glucoside en milieu solide.

Dans le milieu, contenant base Columbia à une concentration de 46,37 g/l, a été ajouté soit :
- de l'Alizarine-2-β-D-glucoside à 0,05 g/l et du Citrate de Fer Ammoniacal à 0,05 g/l,
- de l'Alizarine-1-β-D-glucoside à 0,05 g/l ou 0,1 g/l et, pour ces deux concentrations, du Citrate de Fer Ammoniacal-à 0,05-g/l,
- du 6-Chloro-3-indolyl-β-D-glucoside à 0,15 g/l sans Citrate de Fer Ammoniacal.

Ces quatre milieux ont été répartis en boîtes de Pétri à raison de 20 ml de milieu par boîte. Des micro-organismes ont été ensemencés sur ce milieu par isolement en trois cadrans à partir d'une suspension à 0,5 McFarland. Les boîtes ont été incubées à 37 °C pendant 48 heures. Les colonies formées ont été examinées visuellement après 18, 24 et 48 heures d'incubation. La coloration de ces colonies ainsi que l'intensité de cette coloration ont été notées. Les résultats sont présentés dans le tableau 7 ci-dessous :

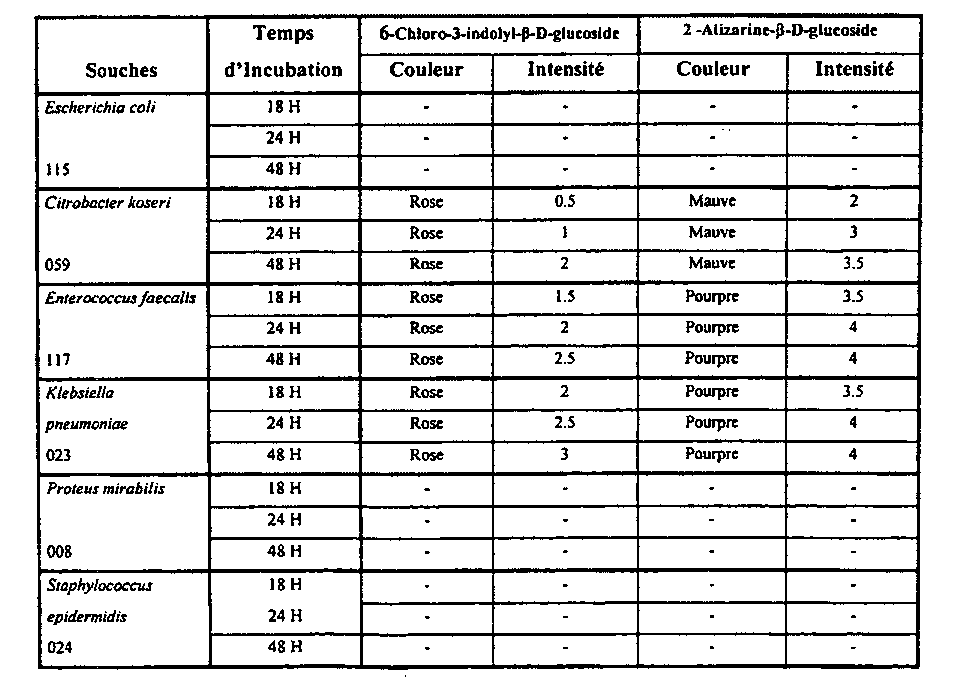

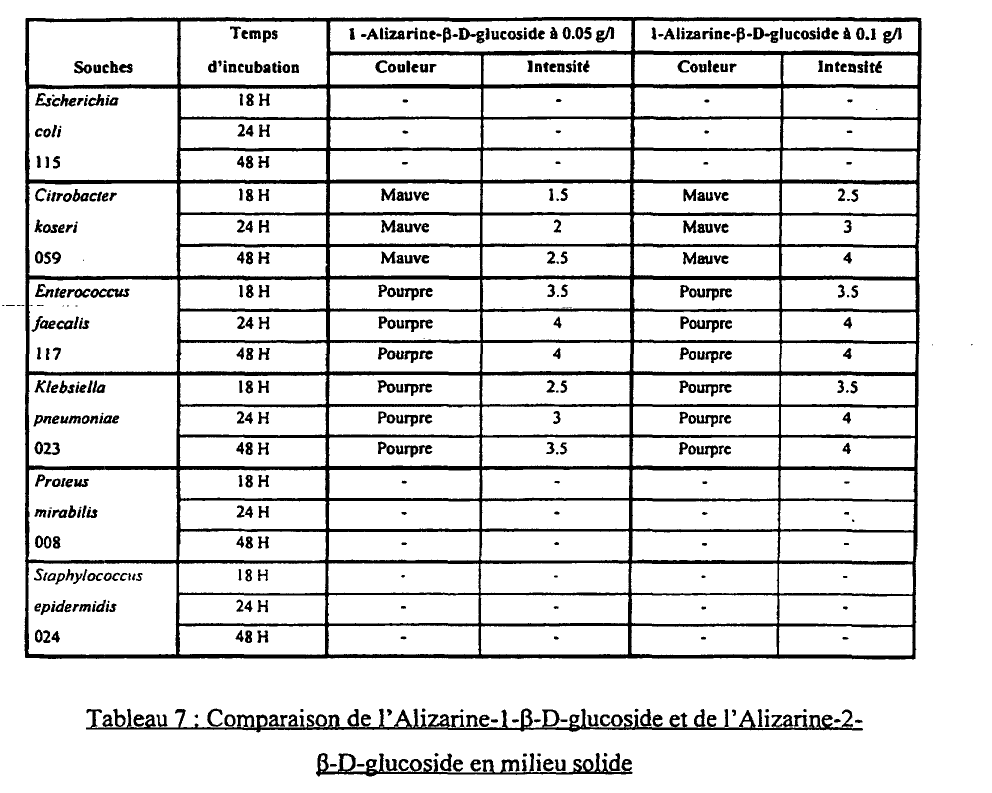

Le symbole - dans ce tableau 7 signifie une absence de coloration. Les souches ainsi testées, n'ayant que des résultats négatifs, font office de contrôle négatif.

A la même concentration 0,05 g/l, les deux substrats ne permettent pas d'obtenir les mêmes intensités de coloration pour deux (2) souches sur trois (3) présentant une activité. L'Alizarine-2-β-D-glucoside est légèrement plus sensible que l'Alizarine-1-β-D-glucoside. Mais à 0,1 g/l l'Alizarine-1-β-D-glucoside permet d'obtenir des intensités de coloration supérieures à celles observées avec l'Alizarine-2-β-D-glucoside.

D'après ce tableau il est aussi possible de remarquer que les intensités obtenues avec l'Alizarine-1-β-D-glucoside à 0,05 g/l sont, pour toutes les souches testées, supérieures à celles observées avec le 6-Chloro-3-indolyl-β-D-glucoside dont la concentration est trois fois supérieure à celle de l'Alizarine-1-β-D-glucoside.

Les substrats à base d'Alizarine sont donc plus sensibles que les substrats à base d'Indoxyl.

Le choix entre l'Alizarine-1-β-D-glucoside et l'Alizarine-2-β-D-glucoside pourra être dicté par d'autres critères, tels que le coût de synthèse, la spécificité dans l'application, la stabilité, etc.

### POINTS PARTICULIERS

Une méthode de visualisation et donc d'identification d'espèces bactériennes, en colonies, s'effectue sur un milieu à base d'agar par incorporation d'au moins un substrat tel que synthétisé ci-dessus en présence de faibles quantités d'au moins un cation idoine. Les colonies révélées forment alors des entités très colorées (rouge, pourpre, bleu, etc.) selon le choix du substrat et du cation qui y est associé. Lorsque ce ratio entre le substrat et le cation existe, il est compris entre 1/100 et 100/1, préférentiellement entre 1/10 et 10/1, et encore plus préférentiellement entre 1/2 et 2/1.

Dans certaines conditions, la présence d'un substrat de SO₃H en R₃ permet d'éviter les problèmes d'instabilité et d'insolubilité liés à ce type de substrats.

Par « cations polyvalents », il faut comprendre cations métalliques multivalents, de forme Xⁿ⁺, avec n = 2, 3 ou 4. Les métaux utilisables sont : Mg, Al, Ca, Ti, V, Cr, Mn, Fe, Co, Ni, Cu, Zn, Sr, Zr, Sn, Sb, Ba, La, Hf, ainsi que les lanthanides Ce, Sm, Eu, Gd et Tb.

Les Anthrarobines, également appelées Désoxyalizarine ou Anthracène-1,2,10-triol, qui sont des produits réduits des Alizarines, sont déjà bien connues de l'homme du métier. Cette réduction s'effectue par l'action d'Hydroxyde de zinc ou de solution ammoniacale, de Chlorure stanneux d'acide, etc. La formule générale de ce composé est la suivante :

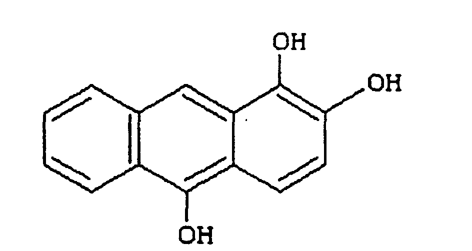

Du fait de l'absence du cycle Quinonoïdal central de l'Anthraquinone, les chélates métalliques du système 1,2-diol ont une réactivité différente, par exemple le chélate ferreux est de couleur noir. Comme le chélate ferreux des Alizarines est de couleur rouge, il est possible d'utiliser deux substrats différents avec le même type de cation, permettant de détecter deux activités enzymatiques différentes. Ces deux substrats peuvent être, par exemple :
- l'Alizarine-2-β-D-glucoside pour détecter une activité osidase, et
- la Désoxyalizarine-2-β-D-phosphatase pour détecter une activité phosphatase.

Il est également possible de protéger le groupement Hydroxyl en position 10 des Anthrarobines, où l'atome d'Hydrogène peut être remplacé par un groupement Méthyl par exemple, ce qui donne une molécule de la forme suivante : 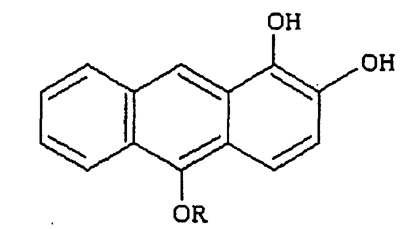 De même, les Alizarines aussi peuvent être protégées en réduisant les cétones sous forme d'alcool 9,10-diol, fonctions alcooliques qui sont ensuite alkylées, par exemple sous la forme de 1,2-Dihydroxy-9,10-diméthoxyvanthracène, comme représenté ci-dessous :

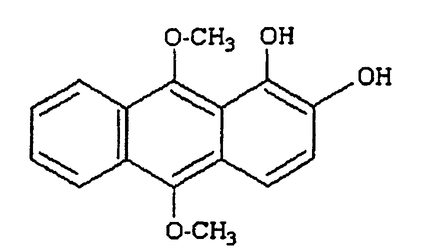

Ces Anthrarobines et Alizarines alkylées ne peuvent ensuite spontanément être transformées en Alizarines par oxydation à l'air libre, et sont de ce fait de bons candidats pour des glycosidations des substrats, préférentiellement en position 2.

## Revendications

1. Utilisation d'un subsbrat chromogène de formule générale : 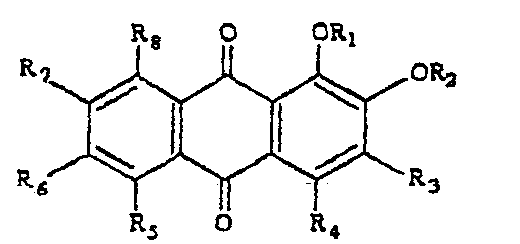 dans laquelle :
- R₁ est une partie cible ou H et R₂ est une partie cible ou H, au moins l'un des R₁ et R₂ étant une partie cible,
- R₃ est H, SO₃H, Cl, Br, F, I, NO₂, NH₂, NR₉R₁₀, Acylamino, Aminoaryl ou Aminoacylamino du type NHCOX, avec X égal à Alkyl, Aryl Aralkyl ou un résidu d'acide α-aminé, tel que l'Alanine,
- R₄ est H, SO₃H, Cl, Br, F, I, NO₂, NH₂, NR₉R₁₀, OH, Acylamino, Aminoaryl ou Aminoacylamino du type NHCOX, avec X égal à Alkyl, Aryl, Aralkyl ou un résidu d'acide α-aminé, tel que l'Alanine,
- selon une variante, R₃ et R₄ sont reliés l'un à l'autre pour former un cycle à au moins cinq côtés, et préférentiellement à six côtés,
- R₅, R₆, R₇ et R₈ sont chacun constitués d'un des atomes ou groupements d'atomes suivants : H, Halogène, particulièrement Cl ou Br, OH, SO₃H, Alkyl ou Alkoxy, et
- R₉ et R₁₀ sont constitués indépendamment de Méthyl, Alkyl, Aryl, Aralkyl ou constituent l'un, R₉ ou R₁₀, un cycle (Pipéridine, Pyrrolidine, Morpholine, etc.) et l'autre, R₁₀ ou R₉, un atome d'Hydrogène,
pour détecter la présence d'une activité enzymatique.

2. Utilisation, selon la revendication 1, **caractérisé par le fait que** les cétones du cycle central sont réduites sous forme de groupements Hydroxyde dans lequel au moins un atome d'hydrogène est éventuellement remplacé par un groupement Méthyl, Alkyl, Aryl, Aralkyl.

3. Utilisation d'un substrat chromogéne de formule générale : 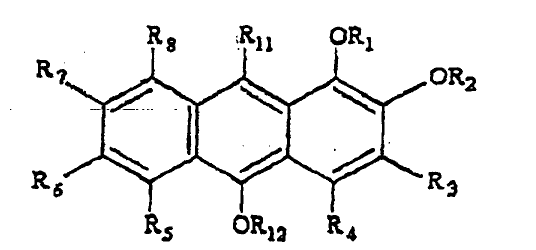 dans laquelle:
- R₁ est une partie cible ou H et R₂ est une partie cible ou H, au moins l'un des R₁ et R₂ étant une partie cible,
- R₃ est H, SO₃H, Cl, Br, F, I, NO₂, NH₂, NR₉R₁₀, Acylamino, Aminoaryl ou Aminoacylamino du type NHCOX, avec X égal à Alkyl, Aryl, Aralkyl ou un résidu d'acide α-aminé, tel que l'Alanine,
- R₄ est H, SO₃H, Cl, Br, F, I, NO₂, NH₂, NR₉R₁₀, OH, Acylamino, Aminoaryl ou Aminoacylamino du type NHCOX, avec X égal à Alkyl, Aryl, Aralkyl on un résidu d'acide α-aminé, tel que l'Alanine,
- selon une variante, R₃ et R₄ sont reliés l'un à l'autre pour former un cycle à au moins cinq côtes, et préférentiellement à six côtés,
- R₅, R₆, R₇ et R₈ sont chacun constitués d'un des atomes ou groupements d'atomes suivants : H, Halogène, particulièrement Cl ou Br, OH, SO₃H, Alkyl ou Alkoxy,
- R₉ et R₁₀ sont constitués indépendamment de Méthyl, Alkyl, Aryl Aralkyl ou constituent l'un, R₉ ou R₁₀, un cycle (Pipéridine, Pyrrolidine, Morpholine, etc.) et l'autre, R₁₀ ou R₉, un atome d'Hydrogène,
- R₁₁ est constitué d'un des atomes ou groupements d'atomes suivants : H, SO₃H, Cl, Br, F, I, NO₂, NH₂, NR₉R₁₀, Alkyl, Aryl, Aralkyl, Acylamino, Aminoaryl ou Aminoacylamino du type NHCOX, avec X égal à Alkyl, Aryl Aralkyl ou un résidu d'acide α-aminé, tel que l'Alanine, et
- R₁₂ est constitué de H, Méthyl, Alkyl, Aryl, Aralkyl,
pour détecter la présence d'une activité enzymatique.

4. Utilisation, selon l'une quelconque des revendications 1 à 3, **caractérisé par le fait que** R₁ est H et R₂ est une partie cible.

5. Utilisation, selon l'une quelconque des revendications 1 à 4, **caractérisé par le fait que** la partie cible est constituée par l'une des molécules suivantes :
- un Glycoside, constitué par des unités mono-, di- et/ou polysaccharides, liés en α ou β à la fonction Hydroxyle,
- un acide α-aminé ou un peptide,
- un acide organique, tel que -O-CO-(CH₂)ₙ-CH₃, avec n compris entre 0 et 20, ou
- un Sulfate, un Phosphate, un Pyrosulfate, un Pyrophosphate ou un Phosphodiester.

6. Utilisation, selon l'une quelconque des revendications 1 à 5, **caractérisé par le fait que** R₃ et R₄ sont constitués et reliés l'un à l'autre par une chaîne en C₃N, substituée ou non, avec préférentiellement N adjacent de R₃ ou de R₄, afin de former un cycle à six côtés.

7. Utilisation d'au moins un substrat, selon l'une quelconque des revendications 1 à 6, pour permettre de détecter la présence d'une activité enzymatique, caractérisée en ce qu'elle consiste :
- à mettre au moins un substrat, dont la partie cible est en relation avec l'activité enzymatique à détecter, en présence d'un échantillon suspecté de contenir au moins un micro-organisme ayant une telle activité enzymatique, et d'au moins un type de cation, et
- à observer la formation d'un chélate insoluble et coloré.

8. Utilisation, selon la revendication 7, **caractérisée en ce que** le substrat est mis en présence d'au moins un type de cation approprié par rapport à la partie marqueur libérée par l'activité enzymatique.

9. Utilisation, selon l'une quelconque des revendications 7 ou 8, **caractérisée en ce qu'**un type de cation, qui peut être utilisé pour former un chélate insoluble, est constitué par : Fe²⁺, Al³⁺, Mn²⁺, Sn²⁺ ou Cu²⁺.

10. Utilisation d'au moins deux substrats, selon l'une quelconque des revendications 1 à 6, pour permettre de détecter la présence d'au moins deux activités enzymatiques différentes, caractérisée en ce qu'elle consiste :
- à mettre au moins deux substrats, dont les parties cibles sont en relation avec les au moins deux activités enzymatiques à détecter, en présence d'un échantillon suspecté de contenir au moins un micro-organisme ayant de telles activités enzymatiques, et d'au moins un type de cation, et
- à observer la formation d'au moins deux colorations différentes ou d'une tierce coloration.

11. Utilisation, selon la revendication 10, **caractérisée en ce que** les substrats sont mis en présence d'au moins un type de cation, préférentiellement d'un seul type de cation, approprié par rapport aux parties marqueurs libérées par les activités enzymatiques.

12. Utilisation d'au moins un substrat, selon l'une quelconque des revendications 1 à 6, pour permettre de détecter la présence d'une activité enzymatique, ou en combinaison avec une utilisation selon l'une quelconque des revendications 7 à 11, caractérisée en ce qu'elle consiste:
- à mettre au moins un substrat, dont la partie cible est en relation avec l'activité enzymatique à détecter, en présence d'un échantillon suspecté do contenir au moins un micro-organisme ayant une telle activité enzymatique, dans un milieu réactionnel ayant un pH approprié, et
- à observer la formation d'au moins une coloration.

13. Utilisation, selon l'une quelconque des revendications 7 à 12, **caractérisée en ce que** l'utilisation d'au moins deux substrats entraîne l'utilisation d'un seul type de cation, approprié par rapport aux parties marqueurs libérées par les activités enzymatiques.

14. Utilisation, selon l'une quelconque des revendications 7 ou 10, **caractérisée en ce qu'**elle consiste à effectuer une étape intermédiaire consistant à laisser pousser le ou les micro-organismes sur un milieu de culture contenant le ou les substrats.

15. Utilisation, selon l'une quelconque des revendications 7 à 14, **caractérisée en ce que** l'on détecte une activité enzymatique glycosidase en utilisant, entre autres, comme partie cible :
• Glucose,
• Galactose,
• Mannose,
• Xylose,
• Acide glucuronique, ou
• N-Acétylglucosamine.

16. Utilisation, selon l'une quelconque des revendications 7 à 14, **caractérisée en ce que** l'on détecte une activité enzymatique phosphatase en utilisant comme partie cible, entre autres, l'Acide phosphorique ou un dérivé substitué.

17. Utilisation, selon l'une quelconque des revendications 7 à 14, **caractérisée en ce que** l'on détecte une activité enzymatique sulfatase en utilisant comme partie cible, entre autres, l'Acide sulfurique ou un dérivé substitué.

18. Utilisation, selon l'une quelconque des revendications 7 à 14, **caractérisée en ce que** l'on détecte une activité enzymatique lipase, phospholipase ou estérase en utilisant comme partie cible, entre autres :
• un acide gras, saturé ou non, ou un dérivé substitué,
• l'Acide acétique ou un dérivé substitué,
• l'Acide butyrique ou un dérivé substitue.
• l'Acide octanoïque ou un dérivé substitué, ou
• un groupement Phosphate estérifié, tel que l'Inositol-1-phosphate.

19. Composition permettant la détection d'au moins une souche et/ou une espèce de micro-organismes qui comprend au moins un substrat, selon l'une quelconque des revendications 1 à 6, et un milieu de culture.

20. Composition, selon la revendication 19, **caractérisée par** le fait qu'elle comporte au moins deux substrats, les substrats, après réaction, sont de couleurs différentes permettant de différencier différentes activités enzymatiques révélées d'au moins une souche et/ou une espèce de micro-organismes.

21. Composition, selon l'une quelconque des revendications 19 ou 20, **caractérisée par le fait qu'**elle est constituée par un milieu de culture liquide, gélifié ou solide.

22. Composition, selon l'une quelconque des revendications 19 à 21, **caractérisée par le fait que** la concentration du substrat est comprise entre 10 et 500 mg/l, préférentiellement entre 30 et 150 mg/l, et encore plus préférentiellement est de 50 mg/l.

## Patentansprüche

1. Eine Verwendung eines chromogenen Substrats mit der allgemeinen Formel: 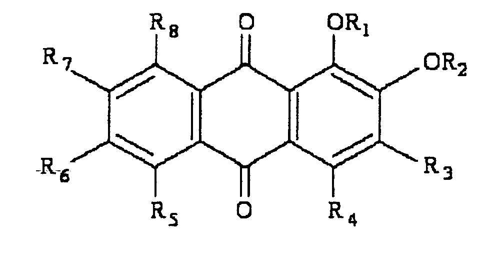 wobei:
- R₁ ein Zielteil oder H ist und R₂ ein Zielteil oder H ist, wobei mindestens R₁ oder R₂ ein Zielteil ist,
- R₃ H, SO₃H, Cl, Br, F, l, NO₂, NH₂, NR₉R₁₀, Acylamin, Aminoaryl oder Aminoacylamin des Typs NHCOX ist, wobei X gleich Alkyl, Aryl Aralkyl oder einem α-Aminsäurerückstand, wie Alanin, ist,
- R₄ H, SO₃H, Cl, Br, F, I, NO₂, NH₂, NR₉R₁₀, OH, Acylamin, Aminoaryl oder Aminoacylamin des Typs NHCOX ist, wobei X gleich Alkyl, Aryl, Aralkyl oder einem α-Aminsäurerückstand, wie Alanin, ist,
- R₃ und R₄ gemäß einer Variante miteinander verbunden sind, um einen Zyklus zu bilden, der mindestens fünf Seiten und vorzugsweise sechs Seiten aufweist,
- R₅, R₆, R₇ und R₈ jeweils aus einem der folgenden Atome oder einer der folgenden Atomgruppen zusammengesetzt sind: H, Halogen, insbesondere Cl oder Br, OH, SO₃H, Alkyl oder Alkoxy, und
- R₉ und R₁₀ unabhängig aus Methyl, Alkyl, Aryl, Aralkyl zusammengesetzt sind, oder eines, R₉ oder R₁₀, aus einem Zyklus (Piperidin, Pyrrolidin, Morpholin etc.) zusammengesetzt ist, und das andere, R₁₀ oder R₉, aus einem Wasserstoffatom zusammengesetzt ist,
um die Anwesenheit einer enzymatischen Aktivität zu ermitteln.

2. Verwendung gemäß Anspruch 1, **dadurch gekennzeichnet, dass** die Ketone des mittleren Zyklus in Form von Hydroxidgruppen reduziert werden, wobei mindestens ein Wasserstoffatom letztendlich durch eine Gruppe aus Methyl, Alkyl, Aryl, Aralkyl ersetzt wird.

3. Eine Verwendung eines chromogenen Substrats mit der allgemeinen Formel: 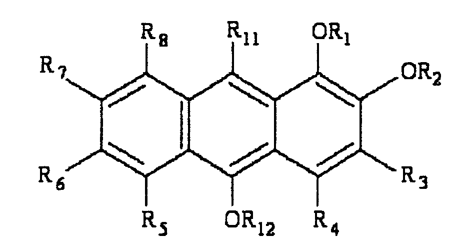 wobei:
- R₁ ein Zielteil oder H ist und R₂ ein Zielteil oder H ist, wobei mindestens R₁ oder R₂ ein Zielteil ist,
- R₃ H, SO₃H, Cl, Br, F, I, NO₂, NH₂, NR₉R₁₀, Acylamin, Aminoaryl oder Aminoacylamin des Typs NHCOX ist, wobei X gleich Alkyl, Aryl Aralkyl oder einem α-Aminsäurerückstand, wie Alanin, ist,
- R₄ H, SO₃H, Cl, Br, F, I, NO₂, NH₂, NR₉R₁₀, OH, Acylamin, Aminoaryl oder Aminoacylamin des Typs NHCOX ist, wobei X gleich Alkyl, Aryl, Aralkyl oder einem α-Aminsäurerückstand, wie Alanin, ist,
- R₃ und R₄ gemäß einer Variante miteinander verbunden sind, um einen Zyklus zu bilden, der mindestens fünf Seiten und vorzugsweise sechs Seiten aufweist,
- R₅, R₆, R₇ und R₈ jeweils aus einem der folgenden Atome oder einer der folgenden Atomgruppen zusammengesetzt sind: H, Halogen, insbesondere Cl oder Br, OH, SO₃H, Alkyl oder Alkoxy,
- R₉ und R₁₀ unabhängig aus Methyl, Alkyl, Aryl, Aralkyl zusammengesetzt sind, oder eines, R₉ oder R₁₀, aus einem Zyklus (Piperidin, Pyrrolidin, Morpholin etc.) zusammengesetzt ist und das andere, R₁₀ oder R₉, aus einem Wasserstoffatom zusammengesetzt ist,
- R₁₁ aus einem der folgenden Atome oder einer der folgenden Atomgruppen zusammengesetzt ist: H, SO₃H, Cl, Br, F, I, NO₂, NH₂, NR₉R₁₀, Alkyl, Aryl, Aralkyl, Acylamin, Aminoaryl oder Aminoacylamin des Typs NHCOX, wobei X gleich Alkyl, Aryl, Aralkyl oder einem α-Aminsäurerückstand, wie Alanin, ist, und
- R₁₂ aus H, Methyl, Alkyl, Aryl, Aralkyl zusammengesetzt ist,
um die Anwesenheit einer enzymatischen Aktivität zu ermitteln.

4. Verwendung gemäß einem der Ansprüche 1 bis 3, **dadurch gekennzeichnet, dass** R₁ H ist und R₂ ein Zielteil ist.

5. Verwendung gemäß einem der Ansprüche 1 bis 4, **dadurch gekennzeichnet, dass** der Zielteil aus einem der folgenden Moleküle zusammengesetzt ist:
- einem Glykosid, das aus Mono-, Di- und/oder Polysaccharideinheiten zusammengesetzt ist, die durch α oder β mit der Hydroxylfunktion verbunden sind,
- einer α-Aminosäure oder einem Peptid,
- einer organischen Säure, wie -O-CO-(CH₂)ₙ-CH₃, wobei n zwischen 0 und 20 liegt, oder
- einem Sulfat, einem Phosphat, einem Pyrosulfat, einem Pyrophosphat oder einem Phosphodiester.

6. Verwendung gemäß einem der Ansprüche 1 bis 5, **dadurch gekennzeichnet, dass** R₃ und R₄ aus einer substituierten oder nicht substituierten C₃N-Kette, wobei N vorzugsweise an R₃ oder R₄ angrenzt, zusammengesetzt und durch diese miteinander verbunden sind, um einen Zyklus mit sechs Seiten zu bilden.

7. Verwendung von mindestens einem Substrat gemäß einem der Ansprüche 1 bis 6, um das Ermitteln der Anwesenheit einer enzymatischen Aktivität zu ermöglichen, **dadurch gekennzeichnet, dass** sie aus Folgendem besteht:
- Zusammenbringen von mindestens einem Substrat, dessen Zielteil mit der zu ermittelnden enzymatischen Aktivität in Verbindung steht, mit einer Probe, von der angenommen wird, dass sie mindestens einen Mikroorganismus mit einer derartigen enzymatischen Aktivität enthält, und mit mindestens einer Kationenart, und
- Beobachten der Bildung eines unlöslichen und farbigen Chelats.

8. Verwendung gemäß Anspruch 7, **dadurch gekennzeichnet, dass** das Substrat mit mindestens einer hinsichtlich des Markerteils, der durch die enzymatische Aktivität freigesetzt wurde, geeigneten Kationart zusammengebracht wird.

9. Verwendung gemäß einem der Ansprüche 7 oder 8, **dadurch gekennzeichnet, dass** die Kationenart, die zum Bilden eines unlöslichen Chelats verwendet werden kann, aus Folgendem zusammengesetzt ist: Fe²⁺, Al³⁺, Mn²⁺, Sn²⁺ oder Cu²⁺.

10. Verwendung von mindestens zwei Substraten gemäß einem der Ansprüche 1 bis 6, um das Ermitteln der Anwesenheit von mindestens zwei unterschiedlichen enzymatischen Aktivitäten zu ermöglichen, **dadurch gekennzeichnet, dass** sie aus Folgendem besteht:
- Zusammenbringen von mindestens zwei Substraten, deren Zielteile mit den mindestens zwei zu ermittelnden enzymatischen Aktivitäten in Verbindung stehen, mit einer Probe, von der angenommen wird, dass sie mindestens einen Mikroorganismus mit derartigen enzymatischen Aktivitäten enthält, und mit mindestens einer Kationart, und
- Beobachten der Bildung von mindestens zwei unterschiedlichen Färbungen oder einer dritten Färbung.

11. Verwendung gemäß Anspruch 10, **dadurch gekennzeichnet, dass** die Substrate mit mindestens einer Kationenart, vorzugsweise einer einzigen Kationart, zusammengebracht werden, die hinsichtlich der Markerteile, die durch die enzymatische Aktivität freigesetzt wurden, geeignet ist.

12. Verwendung von mindestens einem Substrat gemäß einem der Ansprüche 1 bis 6, um das Ermitteln der Anwesenheit einer enzymatischen Aktivität zu ermöglichen, oder in Kombination mit einer Verwendung gemäß einem der Ansprüche 7 bis 11, **dadurch gekennzeichnet, dass** sie aus Folgendem besteht:
- Zusammenbringen von mindestens einem Substrat, dessen Zielteil mit der zu ermittelnden enzymatischen Aktivität in Verbindung steht, mit einer Probe, von der angenommen wird, dass sie mindestens einen Mikroorganismus mit einer derartigen enzymatischen Aktivität enthält, in einem Reaktionsmedium mit einem geeigneten pH-Wert, und
- Beobachten der Bildung mindestens einer Färbung.

13. Verwendung gemäß einem der Ansprüche 7 bis 12, **dadurch gekennzeichnet, dass** die Verwendung von mindestens zwei Substraten die Verwendung einer einzigen Kationenart bedingt, die hinsichtlich der Markerteile, die durch die enzymatischen Aktivitäten freigesetzt wurden, geeignet ist.

14. Verwendung gemäß Anspruch 7 oder 10, **dadurch gekennzeichnet, dass** sie aus dem Durchführen eines intermediären Schritts besteht, der aus dem Ermöglichen des Wachsens des/der Mikroorganismus/Mikroorganismen auf einem Nährmedium, das das/die Substrat(e) enthält, besteht.

15. Verwendung gemäß einem der Ansprüche 7 bis 14, **dadurch gekennzeichnet, dass** die enzymatische Glycosidase-Aktivität ermittelt wird, indem, unter anderem, Folgende als Zielteil verwendet werden:
• Glucose
• Galaktose
• Mannose
• Xylose
• Glucuronsäure, oder
• N-Acetylglucosamin.

16. Verwendung gemäß einem der Ansprüche 7 bis 14, **dadurch gekennzeichnet, dass** eine enzymatische Phosphatase-Aktivität ermittelt wird, indem, unter anderem, Phosphorsäure oder ein substituiertes Derivat als Zielteil verwendet wird.

17. Verwendung gemäß einem der Ansprüche 7 bis 14, **dadurch gekennzeichnet, dass** eine enzymatische Sulfatase-Aktivität ermittelt wird, indem, unter anderem, Schwefelsäure oder ein substituiertes Derivat als Zielteil verwendet wird.

18. Verwendung gemäß einem der Ansprüche 7 bis 14, **dadurch gekennzeichnet, dass** eine enzymatische Lipase-, Phospholipaseoder Esterase-Aktivität ermittelt wird, indem, unter anderem, Folgende als Zielteil verwendet werden:
• eine gesättigte oder ungesättigte Fettsäure, oder ein substituiertes Derivat,
• Essigsäure oder ein substituiertes Derivat,
• Buttersäure oder ein substituiertes Derivat,
• Octansäure oder ein substituiertes Derivat, oder
• eine veresterte Phosphatgruppe, wie Inositol-1-Phosphat.

19. Eine Verbindung zum Ermöglichen des Ermittelns von mindestens einem Mikroorganismenstamm und/oder einer Mikroorganismenspezies, die mindestens ein Substrat gemäß einem der Ansprüche 1 bis 6 und ein Nährmedium umfasst.

20. Verbindung gemäß Anspruch 19, **dadurch gekennzeichnet, dass** sie mindestens zwei Substrate umfasst, wobei die Substrate nach der Reaktion unterschiedliche Farben aufweisen, wodurch das Unterscheiden der offenbarten unterschiedlichen enzymatischen Aktivitäten des mindestens eines Mikroorganismenstamms und/oder der mindestens einen Mikroorganismenspezies ermöglicht wird.

21. Verbindung gemäß einem der Ansprüche 19 bis 20, **dadurch gekennzeichnet, dass** sie aus einem flüssigen, gelierten oder festen Nährmedium zusammengesetzt ist.

22. Verbindung gemäß einem der Ansprüche 19 bis 21, **dadurch gekennzeichnet, dass** die Konzentration des Substrats zwischen 10 und 500 mg/l, vorzugsweise zwischen 30 und 150 mg/l, liegt und am besten 50 mg/l beträgt.

## Claims

1. Use of a chromogenic substrate with the following general formula: 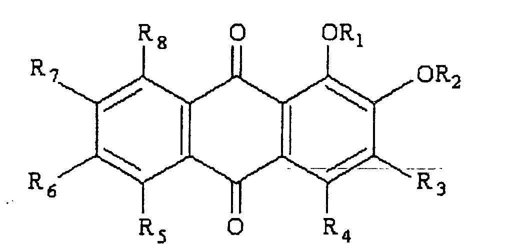 in which:
- R₁ is a target part or H, and R₂ is a target part or H, with at least one out of R₁ and R₂ being a target part,
- R₃ is H, SO₃H, Cl, Br, F, I, NO₂, NH₂, NR₉R₁₀, or an Acylamino Aminoaryl or Aminoacylamino group of the type NHCOX, with X being an Alkyl, Aryl or Aralkyl group or an α-amino acid residue such as Alanine,
- R4 is H, SO₃H, Cl, Br, F, I, NO₂, NH₂, NR₉R₁₀, OH or an Acylamino Aminoaryl or Aminoacylamino group of the type NHCOX, with X being an Alkyl, Aryl or Aralkyl group or an α-amino acid residue such as Alanine,
- according to a modification, R₃ and R₄ form bonds with one another to create a ring with at least five sides, and preferably six sides.
- R₅, R₆, R₇ and R₈ each consist of one of the following atoms or groups of atoms: H, a halogen (particularly Cl or Br), OH, SO₃H, or an Alkyl or Alkoxy group, and
- R₉ a R₁₀ are independently a Methyl, Alkyl, Aryl, Aralkyl group, or one (either R₉ ou R₁₀) is a ring structure (Piperidine, Pyrrolidine, Morpholine, etc.) with the other (either R₁₀ or R₉) being a Hydrogen atom,
for detecting the presence of an enzyme activity.

2. The use, according to Claim 1, **characterized in that** the ketone groups of the central ring are reduced to form hydroxide groups in which at least one of the hydrogen atoms might be replaced by a Methyl, Alkyl, Aryl or Aralkyl group.

3. Use of a chromogenic substrate with the following general formula: 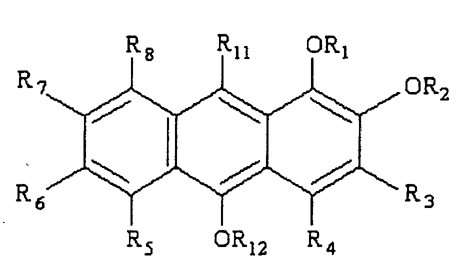 in which:
- R₁ is a target part or H, and R₂ is a target part or H, with at least one out of R₁ and R₂ being a target part,
- R₃ is H, SO₃H, Cl, Br, F, I, NO₂, NH₂, NR₉R₁₀, or an Acylamino Aminoaryl or Aminoacylamino group of the type NHCOX, with X being an Alkyl, Aryl or Aralkyl group or an α-amino acid residue such as Alanine,
- R4 is H, SO₃H, Cl, Br, F, I, NO₂, NH₂, NR₉R₁₀, OH or an Acylamino Aminoaryl or Aminoacylamino group of the type NHCOX, with X being an Alkyl, Aryl or Aralkyl group or an α-amino acid residue such as Alanine,
- according to a modification, R₃ and R₄ form bonds with one another to create a ring with at least five sides, and preferably six sides.
- R₅, R₆, R₇ and R₈ each consist of one of the following atoms or groups of atoms: H, a halogen (particularly Cl or Br), OH, SO₃H, or an Alkyl or Alkoxy group, and
- R₉ a R₁₀ are independently a Methyl, Alkyl, Aryl, Aralkyl group, or one (either R₉ ou R₁₀) is a ring structure (Piperidine, Pyrrolidine, Morpholine, etc.) with the other (either R₁₀ or R₉) being a Hydrogen atom.
- R₁₁ consists of one of the following atoms or groups of atoms: - H, SO₃H, Cl, Br, F, I, NO₂, NH₂, NR₉R₁₀, or an Alkyl, Aryl, Aralkyl, Acylamino Aminoaryl or Aminoacylamino group of the type NHCOX, with X being an Alkyl, Aryl or Aralkyl group or an α-amino acid residue such as Alanine, and
- R₁₂ consists of H, or a Methyl, Alkyl, Aryl or Aralkyl group,
for detecting the presence of an enzyme activity.

4. The use according to any of Claims 1 through 3, **characterized in that** R₁ is H and R₂ is a target part.

5. The use according to any of Claims 1 through 4, **characterized in that** the target part consists of one of the following compounds:
- a Glycoside, consisting of mono-, di- or poly-saccharide sub-units, joined to the hydroxyl group through α or β,
- an α-amino acid or a peptide,
- an organic acid, such as -O-CO-(CH₂)ₙ-CH₃, in which n has a value of between 0 and 20, or
- Sulfate, Phosphate, Pyrosulfate, Pyrophosphate or Phosphodiester.

6. The use according to any of Claims 1 through 5, **characterized in that** R₃ and R₄ are such that, when joined to one another through a C₃N chain (which might or might not be substituted) with the N preferably adjacent to R₃ or R₄, a six-sided ring is formed.

7. The use of at least one substrate according to any of Claims 1 through 6, to detect the presence of an enzyme activity, **characterized in that** it consists in:
- bringing at least one substrate, the target part of which matches the activity of the enzyme being assayed, into contact with a sample suspected of containing at least one microorganism which expresses the enzyme activity in question, and at least one type of cation, and
- monitoring for the formation of an insoluble, colored chelation complex.

8. The use, according to Claim 7, **characterized in that** the substrate is introduced into the presence of at least one type of cation which is appropriate for the marker part released by the enzyme activity.

9. The use, according to either of Claims 7 or 8, **characterized in that** a type of cation, which can be used to form an insoluble chelation complex, consists of: Fe²⁺, Al³⁺, Mn²⁺, Sn²⁺ or Cu²⁺.

10. The use of at least two substrates according to any of Claims 1 through 6, to detect the presence of at least two different enzyme activities, **characterized in that** it consists in:
- bringing at least two substrates, the target parts of which match the activities of the two (at least) enzymes being assayed, into contact with a sample suspected of containing at least one microorganism which expresses the enzyme activities in question, plus at least one type of cation, and
- monitoring for the development of at least two different colors or a third color.

11. The use, according to Claim 10, **characterized in that** the substrates are introduced into the presence of at least one type of cation, preferably a single type of cation, which is appropriate for all the marker parts released by the enzyme activities.

12. The use of at least one substrate according to any of Claims 1 through 6, to detect the presence of an enzyme activity, or in combination with a use according to any of Claims 7 through 11, **characterized in that** it consists in:
- bringing at least one substrate, the target part of which matches the activity of the enzyme being assayed, into contact with a sample suspected of containing at least one microorganism which expresses the enzyme activity in question, in a reaction medium with an appropriate pH, and
- monitoring for the development of at least one color.

13. The use according to any of Claims 7 through 12, **characterized in that** the use of at least two substrates involves the use of just one type of cation which is appropriate for all the marker parts released by the enzyme activities.

14. The use according to any of Claims 7 through 10, **characterized in that** it consists in performing an intermediate step involving the growth of the microorganism(s) on a culture medium supplemented with the substrate(s).

15. The use according to any of Claims 7 through 10, **characterized in that** glycosidase enzyme activity is detected, using one of the following (among other possibilities) as the target part:
• Glucose,
• Galactose,
• Mannose,
• Xylose,
• Glucuronic acid, or
• N-acetylglucosamine.

16. The use according to any of Claims 7 through 14, **characterized in that** phosphatase enzyme activity is detected, using Phosphoric acid or a derivative thereof (among other possibilities) as the target part.

17. The use according to any of Claims 7 through 14, **characterized in that** sulfatase enzyme activity is detected, using sulfuric acid or a derivative thereof (among other possibilities) as the target part.

18. The use according to any of Claims 7 through 14, **characterized in that** lipase, phospholipase or esterase enzyme activity is detected, using one of the following (among other possibilities) as the target part:
• a fatty acid (saturated or non-saturated), or a substituted derivative thereof,
• Acetic acid, or a substituted derivative thereof,
• Butyric acid, or a substituted derivative thereof,
• Octanoic acid, or a substituted derivative thereof, or
• an esterified phosphate group such as inositol-1-phosphate.

19. A formulation to detect at least one strain and/or species of microorganism which includes at least one substrate according to any of Claims 1 through 6, and a culture medium.

20. The formulation, according to Claim 19, **characterized in that** it contains at least two substrates which give reaction products of different colors so that distinction can be made between different enzyme activities expressed by at least one strain and/or species of microorganism.

21. The formulation according to either of Claims 19 or 20, **characterized in that** it consists of a culture medium which is either liquid, semi-solid or solid.

22. The formulation according to either of Claims 19 or 20, **characterized in that** the substrate is at a concentration of between 10 and 500 mg/l, preferably between 30 and 150 mg/l, and more preferably still 50 mg/l.
